# EUROPEAN PATENT APPLICATION

(11) **EP 4 292 656 A1**
(43) Date of publication of application: **20.12.2023**
(21) Application number: 22186740.1
(22) Date of filing: 25.07.2022
(51) Int. Cl.: A61P 31/00, A61P 31/04, A61P 31/12, A61K 47/69, C07F 5/00

(54) **FULLERENE GALLIUM PHOSPHONATE AND METHODS**

(30) Priority: 17.06.2022 US 202217843703
(71) Applicant: Exothule Inc., Irvine, CA 92653 (US)
(72) Inventor: Butzloff, Peter, Boise, ID 83702 (US)
(74) Representative: Mollekopf, Gerd Willi

(57) **Abstract**

An antimicrobial composition of buckminsterfullerene with gallium phosphonate functional groups (520) is provided to disassemble or make virus particles (540) inert, and to inhibit viral and fungal proteases using catalytic desulfurization. This composition (520) prevents and inactivates novel corona viruses including variant strains of SARS-Cov-2, fungal pathologies such as valley fever, respiratory ailments such as chronic obstructive pulmonary disorder (COPD), pneumonia, and arthropod vectored bacterial pathogens such as Lyme disease. The antiviral properties further prevent conditions leading to uncontrolled cellular proliferation, neoplasms, degenerative malignancy, cancers, and chronic inflammatory diseases. The gallium similarity to iron permits the treatment of osteoporosis, hypertension, and thrombosis by depolymerizing fibrin in the dissolution of clots and restoration of vascular plasticity. The composition (520) can be produced at low temperatures through reactive shear milling. Delivery methods include ingestion, topical application, inhalation, or injection when used as a medicament or as a food supplement.

## Description

### BACKGROUND

### 1. FIELD OF INVENTION

The present invention relates to antimicrobial compositions and methods for making antimicrobial compositions.

### 2. BACKGROUND ART

Heparin and heparin related compounds are used to prevent thrombosis because of their ability to reduce thrombin activity. Stroke, being one possible outcome of thrombosis, is the result of the intracerebral formation of fibrin clots and may lead to irreversible brain damage. Heart tissue damage and vascular blockages are other possible outcomes. State of the art thrombolytic therapies to remove fibrin clots utilize a variety of natural or recombinant plasminogen activators. These activators of fibrinogen cause degradation of cerebral thrombi or fibrin clots and are also used for similar effect in the treatment of heart attack patients. However, when such therapies are administered 4 to 6 hours after the onset of a stroke or heart attack, they fail to remove the blockages in time to rescue damaged tissues from a lack of oxygen and blood supply. Medical evidence by electron microscopy supports the role of excess iron ions in human blood to dramatically enhance the formation of fibrin fibers with thrombin. This provides clear evidence of an iron dysfunction that significantly delays fibrinolysis (breakup of fibrin) in blood clots. Thus, it is well known that iron ions cause the mats of fibrin deposits bonded with blood cells observed in the plasma of patients after heart attacks and strokes.

However, many other causes of dysfunctional blood clotting and thickening that can be mediated by thrombin are possible. One recent concern is the stickiness enhancement and thrombosis of blood because of viral spike glycoproteins arising from recurrent exposures to new variants of SARS-COV-2 and those endogenously generated spike glycoproteins formed as an unintended long-term consequence of vaccination by some types of mRNA type of vaccines. Such vaccines are known to confer short-term protection against specific and severe COVID-19 virus variants. Heparin and other anti-thrombosis medicaments have limited or no ability to prevent or treat thrombosis associated with COVID-19 variants, systolic hypertension, or general hypertension. It is now well understood that viral spike glycoproteins, when presented by pathogenic invasions or by prophylactic mRNA induction, serve to worsen the systolic hypertension associated with hardening of the vasculature in aged persons, leading to a significant increase in mortality. It is increasingly clear from epidemiology that the accrual of micro clots from multiple stressors including iron dysregulation may lead to stroke, neurological damage, kidney disease, and heart-attacks.

Gallium metal is slow to dissolve in water at room temperature because it generates a surface oxide film that passivates the bulk from chemical interactions. The application of ultrasound at elevated temperatures facilitates destruction of the gallium surface passivation film to allow reactivity with water or hydroxyl groups provided that OH- ions are present to form Ga2O3, which has excellent surfactant or wetting properties between hydrophobic and hydrophilic materials.

The application of gallium as Ga3+ in medical chemistry arises from the similarity in chemistry with that with Fe3+, where both follow the same biological chemical pathway in proteins such as transferrin where Ga3+ binds, however at 300 times less effectively than Fe3+. This lower bonding affinity of gallium may explain why 69% is excreted in urine after 24 hours, and 91% of gallium is excreted in 48 hours. Gallium also bonds to other organic molecules with an affinity for iron because of a similar ionic radius, electric charge, and ligand coordination as iron. However, once divalent gallium or Ga2+ becomes trivalent gallium or Ga3+, it cannot biologically return to Ga2+, unlike iron, wherein the Fe2+ and Fe3+ are biologically reversible.

Gallium nitrate has been found to be both anti-inflammatory and antimicrobial. It has been used to suppress inflammatory Lupus and arthritis autoimmune responses. When gallium is combined with hydroxyapatite (a biological form of calcium phosphate) or with complex sugars (called polysaccharides) it was found to be significantly anti-fungal and anti-bacterial.

The onset of 'long-covid' is a persistent viral infection in which compromised immunity leads to colonization and inflammation of the brain by various strains of SARS-Cov-2. Also known as covid-19 for the year in which the pandemic started, the evolved strains of this virus in combination with less aggressive endemic viruses such as herpes simplex virus-1 (HSV-1) and commensal zoonotic fungal spores such as *candida albicans,* are now increasingly causing what is commonly known as 'brain fog' and other symptoms. Except for an unusual organic complex of gallium that was reported to inhibit the SARS-COV-2 virus, there are no proactive strategies to eliminate either the symptoms or the associated pathologies for these virally induced conditions in medical or clinical practice.

Of great economic and humanitarian healthcare interest is the use of gallium to combat calcium loss and osteoporosis leading to fragile bones and death from infection on catastrophic bone failure in the population of aged women and in the population of cancer patients exposed to radiation treatments. Gallium serves as a moderate retainer of calcium in osteoporosis to promote bone metabolism. This is because gallium tends to accumulate between the collagen and the calcium phosphate in bone cells by the displacement of iron as Fe3+. Ga3+ is better able to stabilize this interface and therefore slow bone loss. Unfortunately, the use of gallium nitrate needs to be administered by continuous intravenous drip which is uncomfortable and inconvenient to patients. This mechanism is different than that of administering bis-phosphonates, which are also used to retard bone loss effectively without resorting to an IV drip. Both methods are effective to retain calcium. Unfortunately, there are no known studies to possibly combine these two treatments. Gallium phosphonates have never been envisioned or considered for possible synergy to combat osteoporosis. There have been, however, scientific research calls for a detailed comparison between the gallium nitride treatment method and the bis-phosphonate treatment method for osteoporosis mitigation.

Particles of gallium aluminum nitride have been engineered to express a negative surface charge capable of retaining positively charged calcium (Ca2+) cations in a successful reversal of osteoporosis by means of the attachment and spreading of osteogenic mesenchymal bone stem cells, demonstrating that the negatively charged voltage differential at the external surface of these particles is important to the gallium mediated treatment process. However, aluminum nitrides of gallium have side effects that preclude these substances from being of long-term use to human patients, leaving the further development of a potential gallium mediated treatment for osteoporosis unexplored. There is, however, medical consensus that making use of a negative electric charge field around a positively charged gallium ion will be desirable if it can be achieved without introducing the toxic side effects from adjunctive structural materials such as aluminum nitrides.

Some compounds of gallium can also be used as a therapeutic agent in cancer. The US National Cancer Institute has found that gallium compounds localize in the same locations as iron in tumors and cancers, and this has led to the use of gallium nitrate as a drug to suppress tumors under NSC 15200 in the treatment of non-Hodgkin's lymphoma and bladder cancer. Gallium nitrate binds as transferrin-Ga3+ and stops DNA replication by depriving ribonucleotide reductase of iron by its presence, leading to cancer cell death while leaving healthy cells intact. Phenolic-gallium was used to treat prostate cancer proteins in the form of a 4, 6 phenol-gallium. Gallium maltolate inhibits liver carcinoma cell growth and induces apoptosis in these cells better than gallium nitrate. The medical community urgently awaits the development of additional types of gallium compounds to try in preclinical and clinical testing.

Gallium quantum dots have been explored using ultrasonic actuation of amorphous carbon in water solution, resulting in a carbon core with a gallium oxide shell. Further explorations of gallium found that the attraction was associative, especially when using an ultrasonic treatment, however these efforts failed in producing covalent bonds between C60 and gallium quantum dots. Interestingly, it was found effective to capture the gallium inside the fullerene during the fullerene formation process, but this was still not a bonding type of interaction.

Covalent bonding of phosphonates of fullerene and ruthenium complexes have been reported. This gives credibility to the ability of fullerene phosphonates to form a stable metal bond through the intermediate atoms of oxygen. Fullerene ruthenium phosphonates are reported to be useful for solar energy generation devices but are not known to be useful in a medical or a clinical use for patients or to treat any disease.

The state-of-the-art antiviral compounds and compositions used in medical practice have been directed at the exterior proteins and surfaces of the protective protein shell of latent virus particles, or at the inhibition of the digestive proteases of the active molecular machinery of such viruses. For the most part, fungal species and bacteria often host viral particles, leading to synergy between these types of microbes. It is difficult to combat the virus inside one of these organisms when one of them is pathogenic and hidden within the other.

The number of gallium phosphonate compounds known at present is still very rare. Gallium phosphonate ligands have been explored that contain a pyridine organic functional group, or a tertiary butyl group. These gallium phosphonates were developed for the purpose of making crystalline cage shaped structures. Phosphonates are generally known to be antimicrobial, as are fullerenes such as buckminsterfullerene (C60), and antimicrobial properties have been reported for gallium hydroxides in water as well. Interestingly, the literature indicates each of these three types of substances are independently able to act in an anti-cancer manner. Also, there is evidence that any one of these three substances has some ability to stimulate neural growth and neural regeneration. The direct association of gallium with fullerenes was demonstrated to be a loose and transient association. However, an intermediate combination of gallium and fullerene using phosphonates in a combined molecular structure has never been considered from a modelling perspective, or an experimental synthesis. Therefore, nothing is currently known about such matters, except that sufficient scientific evidence establishes that any two of these substances can indeed react with each other.

What is therefore needed is a novel therapeutic strategy or unique material composition used to confer microbial protection in advance to broadly protect against evolving and future pathogens that may help prevent and treat cancers and microbial infections. Such a substance should also be effective against virally induced genetic predispositions as environmental triggers for activating the development of cancers and neoplasms. Desirably, such an antimicrobial anticancer treatment should be able to cross the blood-brain-barrier (BBB) to confer prophylactic maintenance or enhancement of cognitive function by boosting immunity and treating systolic hypertension well into old age. It is believed the present invention provides a multifunctional composition, having a biological nanoparticle design stabilized by phosphonates to confer multiple methods of therapeutic and prophylactic use. The carrier formulations described herein embody exemplary methods of administration for this molecular composition.

### SUMMARY OF THE INVENTION

The invention is defined in claims 1, 10, and 13, respectively. Particular embodiments are set out in the dependent claims.

The present invention is directed to a composition of buckminsterfullerene bonded with phosphonate pendant functional groups (FGP) being characterized by being size constrained and able to donate a gallium cation. The donated gallium ion serves to ion exchange with physiological sodium ions, thereby conferring a function as a nano-surfactant to the remaining fullerene phosphonate. The gallium ion synergistically confers an antimicrobial as well as an antioxidant property to the FGP. The composition of the invention can be used to prevent and treat thrombosis, to prevent or treat chronic respiratory illnesses such as pneumonia, and to help treat an uncontrolled cytokine storm arising from an inflammatory reaction to invasive microbes including viruses, fungi, and some types of infectious bacteria. The fullerene gallium phosphonate also serves to desulfurize fungal and viral proteases for treating microbially induced cognitive decline and to promote preventative health to protect against microbial invasion of neural tissues. The fullerene gallium phosphonates also donate Ga3+ to replace iron in proliferative neoplasms to promote gallium mediated apoptosis of cancer cells. Delivery methods include ingestion, topical application, inhalation, or injection when used as a medicament or as a food supplement to maintain or re-establish benign healthy acquired immune homeostasis.

This specification is directed in part to a composition comprising carbon fullerenes covalently derivatized with phosphonates having an oxidation state of three, where this substance is saponified with gallium atoms. The manufactured fullerene gallium phosphonates (FGP) provide antimicrobial, anti-cancer, anti-osteoporosis, and neurological therapeutics with additional viral and fungal protease inhibiting functions via the phosphonate sulfurization reaction. The donated gallium atoms are slowly displaced by ambient physiological sodium atoms. Both gallium and sodium confer nano-surfactant properties capable of reversibly shuttling between the oxygen groups of the phosphonate and the bare aromatic carbon face of the buckminsterfullerene by means of pi-cation bonding. These properties are useful to remediate thrombosis, blood clots, and treat hypertension. This composition also possesses the free radical scavenging chemical function of fullerenes, a viral capsid and spike protein disassembly function, and a biosurfactant function that couples with a pulmonary surfactant to reduce its viscosity and moderate cytokine and chemokine release. The composition can clear pulmonary surfactant and fibrins away from airway passages and remediate vascular fibrin mediated clotting reactions arising from the innate immune response to viruses such as novel corona viruses and the SARS-COV-2 virus variants. The result of the antimicrobial functions is to allow time for acquired immunity to be established as each new variant of rapidly evolving microbes is dissolved and disassembled for the body to learn a new immune response.

One aspect of the present composition is that the molecular structure of FGP is such that it produces reversible hopping pi-cation bonded adducts between the gallium ions and any ambient sodium cations among the hydrophilic phosphonate groups and the hydrophobic aromatic regions of the C60 carbon. This is the nano-surfactant property which functions as a hydrophobic penetrant to van-der-Waals charge-stabilized viral proteins.

In another aspect, gallium cations are carried by fullerene gallium phosphonates in such a manner that this molecule can cross the fatty membranes of the blood brain barrier to deliver the hydrophilic gallium ionic cargo to the neural structures of the brain. The release of gallium cations then functions to initiate significant neurite growth. This aspect is important for the treatment of traumatic brain injury, and the recovery of damaged neural cells from the effects of any type of neurological pathology.

In another aspect, gallium cations are carried by fullerene gallium phosphonates in such a manner that this molecule can cross the fatty membranes of the myelin sheath around muscular neurons. This delivery of gallium cations to the inner neural structures functions to initiate significant neurite regrowth from physical damage and trauma. The genetic predisposition towards poor recovery from ordinary damage in muscular dystrophies such as Duchenne's muscular dystrophy can be remediated. This aspect is important for the treatment of children with injury from ordinary activities and play to help correct and recover those regions of damaged neural cells from the effects of this type of neurological genetic defect.

In another aspect, the donation of gallium cations serves to remediate and displace available sites for iron to help address Fe3+ dysfunction in a treatment for hypertension.

In another aspect, FGP is used to promote calcium retention and treat osteoporosis by means of interposing between collagen and the calcium phosphate among bone mesenchymal cells.

In a related aspect, FGP achieves interposition between collagen and calcium phosphate among bone mesenchymal cells by the development of an expressed negative surface charge through the loss of protons attached to the terminal hydroxyl group.

In another aspect, the nano-surfactant property of fullerene gallium phosphonate significantly enhances the anticancer and antitumor effects of donated gallium cations by delivering these ions across the waxy deposits formed at the surfaces of tumors and cancer cells.

In another aspect, the loss of gallium and its replacement by sodium cations on the phosphonate groups enable these groups to become charge-attracted to highly negatively charged exterior hydrophilic regions at the edges of viral capsid glycoprotein plates and at the outside of spike glycoproteins as a nano-surfactant material that, synergistically with gallium cations, deconstructs viral structures.

In a related aspect, either gallium or sodium cations with pi-cation bonds to a buckminsterfullerene molecule have sufficiently small dimensions to displace the chloride ion charge pinning regions in viral spike glycoproteins to allow their immediate disassembly.

In a related aspect, the fullerene gallium phosphonate can perform viral protein capsid van-der-Waals charge disruption, thereby functioning to explode and denature virus particles by taking apart their protective protein shells and exposing their viral RNA for removal by the immune system before these viral particles can infect a cell.

In another aspect, the composition of fullerene gallium phosphonate is used to treat COPD by inhalant delivery to the lungs and airways of a patient (human or animal) in need of treatment.

In a related aspect, the fullerene gallium phosphonate composition is used to treat fungal lung infections such as black fungus or valley fever of the lungs and airways, again by inhalant delivery to a patient that is experiencing this type of chronic inflammatory bronchitis.

In another aspect, the gallium phosphonates pendant from the fullerene serve to inhibit viral proteases, as well as to remove their catalytic protein digestion capability, by irreversibly bonding to and extracting the catalytic sulfur atom from the protease. This immediately halts the pathological impact of malignant, cancer-causing viruses to deconstruct, infect, and usurp the function of otherwise healthy somatic cells. This can also immediately halt a pathological fungal invasion of sensitive organs and tissues.

In another aspect, the surfactant properties of the fullerene gallium phosphonate allow it to diffuse throughout the extremely small dimensions of the hydrophobic regions within viruses. Larger molecules such as organic substituted bisphosphonates are unsuitable to fit inside these viral structures.

In a related aspect, the sodium cations that reversibly hop to and from the phosphonate functional groups on partial or complete loss of gallium cations, as well as the generated gallium hydroxides formed from the loss of gallium from fullerene gallium phosphonates, implement the viral disassembly process using van-der-Waals charge disruption.

In another aspect, the core fullerene molecule functions to disassemble detrimental van-der-Waals mediated hydrophobic charge zippers at usurped cell membranes while decorating and defusing them with nano-surfactant deposits that interfere with and halt the function of viral replication platforms. This action coats and destroys the zippering mechanism of viral protein glycoprotein filaments by masking their hydrophobic van-der-Waals mediated charge zippers. This action of de-zippering the viral glycoprotein filaments is achieved by coating their zippering regions with a multiplicity of fullerene gallium phosphonates.

In another aspect, the chelation ability of the fullerene gallium phosphonate allows the molecule to function as a free radical recombination and detoxification center, thereby reducing inflammation and serving to boost innate immunity while reducing the tendency for excessive cytokine storms and chemokines to inflict damage on tissues.

In another aspect, the chelation ability of fullerene gallium phosphonates allows one of more of these molecules to form a complex with polymeric fibrin filaments as a therapeutic drug molecule to lyse and reduce the formation of blood clots.

In a related aspect, the chelation ability of the fullerene group is well known to create a protein corona around itself, enabling the disruption of pathogenic bacterial protective proteins, and unmasking the pathogen for recognition by macrophages and antibodies. Such pathogens are often transmitted (vectored) by insects (arthropods). Simultaneously the provision of the phosphonate functional groups confers nano surfactant properties used as a penetrant to enable the release and deactivation of these entrapped pathogens and to disrupt their protective proteins for their subsequent recognition and disposal by the acquired immune system.

In another aspect, the fullerene gallium phosphonate composition is aerosolized or heated to form a nano aerosol for the purpose of immediate aspirated delivery to the lungs, thereby providing access to the blood system for rapid release of the administered inhalant composition.

In a related aspect, the fullerene gallium phosphonates serve as a viscosity reducing agent that serves to reduce the viscosity of pulmonary surfactant to enable the clearing of the lungs of patients with respiratory disease such as pneumonia of any type.

These and other advantages of the present invention will be further understood and appreciated by those skilled in the art by reference to the following written specifications, claims, and appended drawings.

Some embodiments are described in detail with reference to the related drawings. Additional embodiments, features, and/or advantages will become apparent from the ensuing description or may be learned by practicing the invention. In the drawings, which are not to scale, like numerals refer to like features throughout the description. The following description is not to be taken in a limiting sense but is made merely for describing the general principles of the invention.

### BRIEF DESCRIPTION OF THE DRAWINGS

Preferred embodiments of the invention will now be described, by way of example, with reference to the accompanying drawings, in which:
FIG. 1 illustrates a chemical reaction of phosphorous acid with buckminsterfullerene to synthesize an intermediate fullerene phosphonate nanoparticle molecular structure.
FIG. 2 illustrates the molecular structures of an exemplary dry chemical reaction of fullerene phosphonates with gallium metal to synthesize a fullerene gallium phosphonate (FGP) of Ga(+2) valence.
FIG. 3 illustrates the molecular structures of a wet chemical reaction of FGP having Ga(+2) valence with water to synthesize an FGP having Ga3+ valence.
FIG. 4 illustrates the molecular structures of an ion exchange reaction of FGP with physiological sodium cations to produce free Ga3+ hydroxides and reversible hopping pi-cation bonded sodium adducts conferring nano-surfactant properties to FGP.
FIG. 5 illustrates the FGP viral protein capsid van-der-Waals charge disruption function.
FIG. 6 illustrates an axial side view of the chloride ion extraction from the hydrophobic region of a viral spike glycoprotein via FGP van-der-Waals mediated charge balance disruption.
FIG. 7 illustrates a radial section view of the chloride ion extraction from the hydrophobic region of a viral spike glycoprotein via van-der-Waals mediated charge balance disruption.
FIG. 8 illustrates reconstruction and recovery of zippered cell membranes usurped and repurposed as viral replication platforms.
FIG. 9 illustrates the protease desulfurization reaction with FGP wherein phosphonate groups extract catalytic sulfur from a viral or fungal amino acid.
FIG. 10 illustrates the gallium donor function of FGP to hypoxic cancer cells enabling apoptosis or cell death of proliferative neoplasms.
FIG. 11 illustrates the neurite growth function of FGP to repair and remediate neurological deficits such as Duchenne's muscular dystrophy.
FIG. 12 illustrates exemplary methods to administer formulations to contain and deliver fullerene sodium phosphonate.
FIG. 13 illustrates administered inhalant FGP entering the lungs.
FIG. 14 illustrates the breakup of blood clots and the prevention of thrombosis by the action of fullerene gallium phosphonates, serving also to reduce systolic hypertension.
FIG. 15 illustrates an exemplary method of personal inhalant administration of a nano-aerosol formulation.
FIG. 16 is a flowchart showing steps to prepare vapor inhalant formulations of FGP.
FIG. 17 is a flowchart representation of exemplary scalable methods for making fullerene gallium phosphonates (FGP) formulated for various forms of oral administration.
FIG. 18 is a flowchart showing steps for making FGP in blood plasma for medical injection.
FIG. 19 illustrates alternative bis and tris phosphonate molecular structures for sodium gallium phosphonate.
FIG. 20 is a flowchart showing exemplary steps to prepare bis- and tris-FGP.
FIG. 21 illustrates FGP mediated bone density recovery from osteoporosis.
FIG 22 illustrates antibacterial activity of FGP to treat insect vectored disease.
FIG. 23 illustrates experimental FTIR test data of fullerene phosphonate.
FIG. 24 illustrates experimental FTIR test data of FGP.

### DETAILED DESCRIPTION OF THE INVENTION

The following detailed description, taken in conjunction with the accompanying drawings, is merely exemplary in nature and is not intended to limit the described embodiments or the application and uses of the described embodiments. Any implementation described herein as "exemplary" or "illustrative" is not necessarily to be construed as preferred or advantageous over other implementations.

Furthermore, there is no intention to be bound by any expressed or implied theory presented in the preceding technical field, background, brief summary or the following detailed description. It is also understood that the specific devices, systems, methods, and processes illustrated in the attached drawings, and described in the following specification, are simply exemplary embodiments of the inventive concepts defined in the appended claims that there may be variations to the drawings, steps, methods, or processes, depicted therein without departing from the scope of the invention. All these variations are within the scope of the present invention. Hence, specific structural and functional details disclosed in relation to the exemplary embodiments described herein are not to be interpreted as limiting, but merely as a representative basis for teaching one skilled in the art to variously employ the present embodiments in virtually any appropriate form, and it will be apparent to those skilled in the art that the present invention may be practiced without these specific details.

Various terms used in the following detailed description are provided and included for giving a perspective understanding of the function, operation, and use of the present invention, and such terms are not intended to limit the embodiments, scope, claims, or use of the present invention.

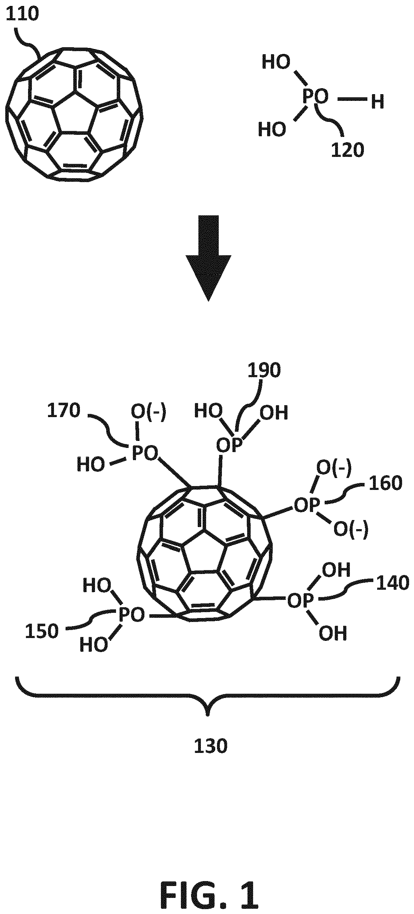

FIG. 1 is an illustration of a chemical reaction to synthesize fullerene phosphonates. Buckminsterfullerene 110 is a molecule in the shape of a spherical chemical cage representing 60 aromatic carbon atoms with formula C60, also known herein as the fullerene molecule. The scientific literature reports a measured single pristine fullerene C60 molecule has a physical particle size about 0.7 nm and a van der Waals diameter of 1.1 nm. The molecular structure of phosphorous acid 120 contains a phosphorous atom with the oxidation state of +3. Fullerene and phosphorous acid are chemically reacted as indicated by the large black arrow to form fullerene phosphonate 130. Alternative phosphonate structures such as bis- and tris- fullerene phosphonates are acceptable replacements for phosphorous acid 120 in the present composition as reactive intermediates.

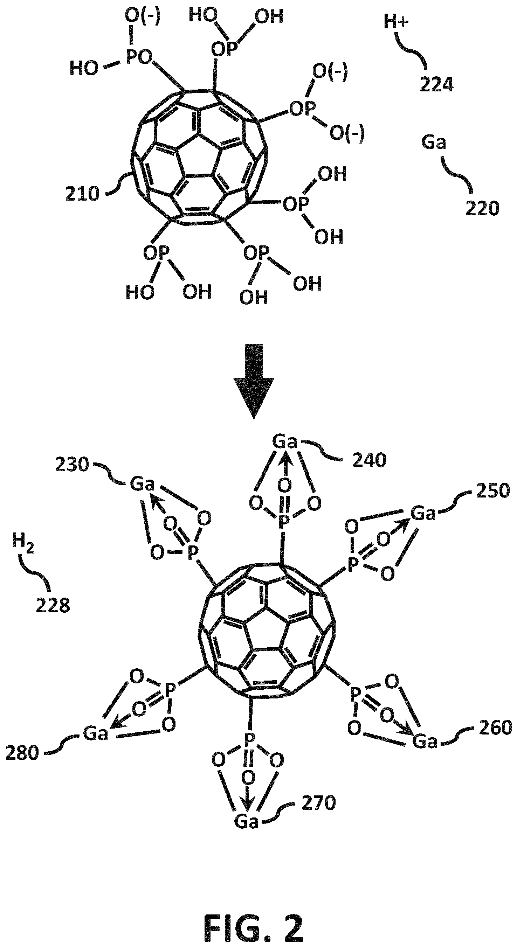

FIG. 2 is an illustration of an exemplary dry chemical reaction of fullerene phosphonates with gallium metal 220 to synthesize an intermediate fullerene gallium phosphonate (FGP) of Ga2+ with the release of hydrogen protons 224. The number of gallium atoms to add in this reaction is determined by the number of phosphonate groups on the fullerene phosphonate. Gallium has an atomic mass of 69.7, whereas the mass of the fullerene phosphonate has a mass sum of 720.7 from fullerene and a mass of 80 for each pendant phosphonate functional group. The preferred addition of gallium metal is stoichiometric with the exact number of phosphonates. It is possible but not desirable to use less gallium which results in reduced efficacy of the prepared FGP. It is possible but not desired to use more gallium than the stoichiometric amount because this leaves unreacted gallium metal microparticles and gallium hydroxides, wherein these microparticles and hydroxides have no ability to cross cell membranes compared with the desired FGP composition.

The FGP reaction can proceed in an air atmosphere containing oxygen because oxygen molecules have no dipole character and are poorly reactive with gallium metal. Gallium metal is a solid at room temperature. It can be added to the reactants at room temperature. Chemical shear reactive processing requires that gallium metal be a liquid to enable the proper dispersion among the other solid reactants. Processing therefore takes place at a temperature at least about 5 °C above 29.8 °C where the gallium metal melts to ensure that liquid-solid wetting takes place. A combination of mechanically induced high shearing and the resulting shear pressure allow the liquid gallium metal to form micro droplets and nanoparticles of gallium atoms that are highly reactive toward the oxygen atoms of the phosphonate groups on fullerene phosphonate, wherein the hydroxyl groups then lose their hydrogen in favor of gallium, which donates electrons to the displaced protons. The displaced protons may then recombine to form molecular hydrogen 228. A multiplicity of small pointing arrows directed at gallium atoms 230, 240, 250, 260, 270, 280 indicate the strong electron withdrawing character of gallium and will be used to convey this aspect of the gallium bonding characteristic arises from the attraction of the lone pair of electrons on phosphorus present in all molecules of fullerene gallium phosphonates hereinafter.

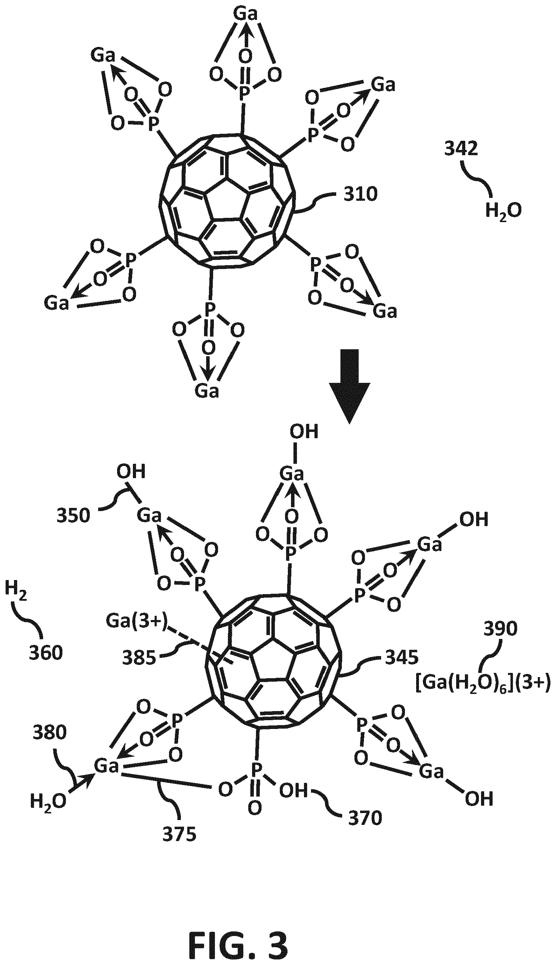

FIG. 3 is an illustration of the molecular structures of a wet chemical reaction of FGP. Fullerene gallium phosphonate molecules 310 are provided as the product of a dry shearing reaction having Ga+2 valence. Molecules of water 342 are then added to initiate the hydrolysis reaction as shown by the direction of the large downward pointing black arrow to synthesize FGP having Ga3+ valence as indicated by the presence of one hydroxyl group pendent from each of a multiplicity of gallium atoms 350 and may release molecular hydrogen 360 during this process. However, the reaction of water may cause the complete hydrolysis of a gallium atom to leave a hydroxyl group at a pendant phosphonate group 370. In related cases, an adjacent gallium atom may form a transient covalent bond to the oxygen atom of a neighboring phosphonate group 375 until another water molecule is drawn by the attraction of its oxygen electrons 380 eventually to also hydrolyze the gallium atom and may release molecular hydrogen during this process. During these reactive processes, gallium cations may associate in pi-cation bonding with the aromatic carbon atoms of the fullerene functional group as indicated by the dashed line 385 or become completely hydrolyzed as the gallium hydroxide or may have a ligand coordination with six water molecules as gallium becomes completely solvated in water 390.

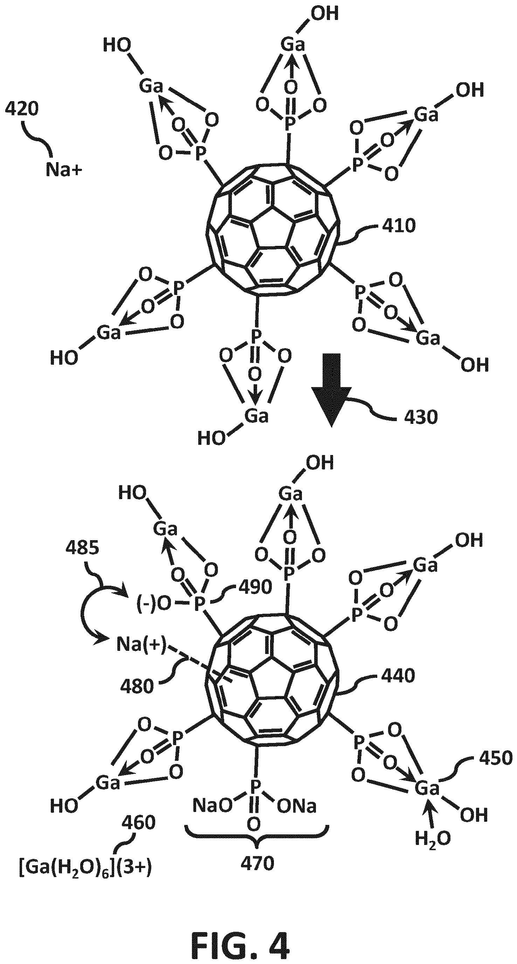

FIG. 4 is an illustration of the molecular structures of an ion exchange reaction of FGP 410 with aqueous physiological sodium cations Na+ 420 which proceeds in the direction of the large black arrow 430 to form fullerene gallium sodium phosphonates 440. The hydrolysis of gallium ions initiates with the lone pair of electrons at the oxygen atom of a water molecule attracted to a gallium atom 450 which is in turn bonded to a phosphonate group. Sufficient hydrolysis releases the gallium atom to release Ga3+ hydroxide molecules that are coordinated to six water molecules 460. In the presence of sufficient physiological sodium cations, the sodium atoms will replace the gallium at the phosphonate group 470. However, some sodium atoms remain as a positive charged ion to from reversible hopping pi-cation bonded sodium adducts with the aromatic carbon atoms of the fullerene functional group as indicated by the dashed line 480. At some of the pi-cation bonded sodium ions will exchange with a proximate gallium phosphonate group as indicated by the double-headed curved arrow 485. It is to be understood that both sodium cations and gallium cations are each able to confer nano-surfactant properties to FGP through phosphonate group 490, according to these teachings.

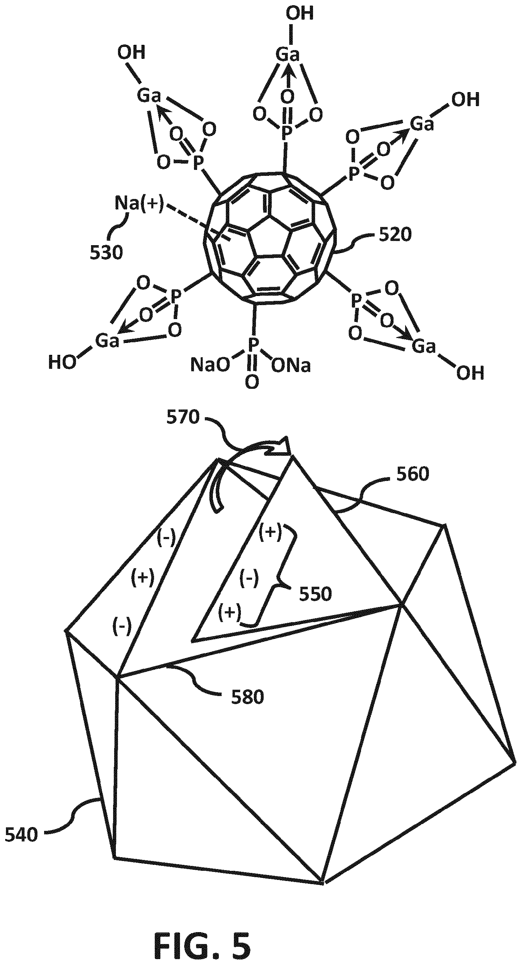

FIG. 5 is an illustration of the FGP viral protein capsid van-der-Waals charge disruption function. Fullerene gallium phosphonate 520, because of its small size, penetrates the symmetric induced charges 550 holding together the assembled protein plates at the hydrophobic regions of the capsid outer shell of a virus particle 540. The presence of ambient physiological sodium cations 530 results in the hopping of some of those sodium cations with a multiplicity of aromatic pi-cation bonds functioning as the nano-surfactant to help penetrate and disrupt the induced charges which hold together the edges of the capsid plates. This results in the disassembling of a first capsid molecular protein plate 560 as shown by the uplifting direction of the large, curved pointing white arrow 570 from the capsid assembly. The region of the opening 580 provides access to further solubilize and remove viral RNA from inside the viral particle capsid assembly 540. The process illustrated here applies to all types of viruses of which strains and types in any configuration or geometry whatsoever are included without limitation, according to these teachings.

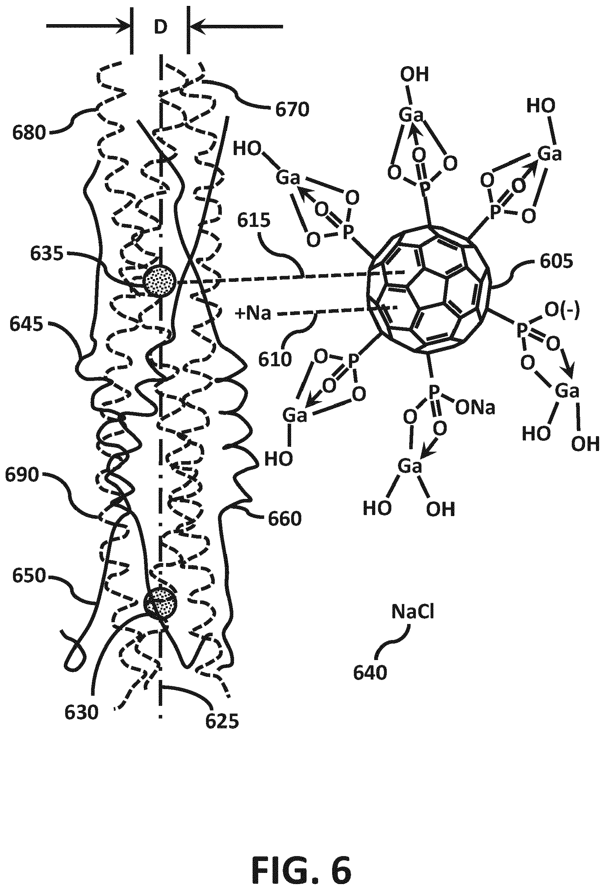

FIG. 6 is an illustration of a side view of the chloride ion extraction from the hydrophobic region of a viral spike glycoprotein via charge balance disruption to destabilize a viral spike glycoprotein of an exemplary influenza or novel coronavirus such as COVID-19 or SARS-Cov-2. Fullerene gallium phosphonate (FGP) 605 is provided with at least one nano-surfactant sodium 610 by ion exchange or that has pi-cation bonded to the aromatic regions of FGP from the ambient physiological saline present in the intercellular environment of the body. FGP mediates the unzippering of the spike glycoprotein outside of a cell membrane to proactively avoid endocytosis. The mechanism to do this is to generate conditions sufficiently like endocytosis that the first step required to unravel the capsid proteins to which it is attached can be performed outside of susceptible cells. Once opened and unraveled, macrophages and other aspects of the immune system can safely dispose of the viral protein fragments including the exposed viral RNA.

The FGP molecule is of sufficiently small size to be capable of accessing the region where negatively charged chloride ions stabilize some types of virus particles or their structures to enable the formation of an aromatic pi-anion bond between the fullerene group and the chloride ion as indicated by a dashed line 615. Centrally located to the viral spike glycoproteins are multiplicity of chloride ions which are positioned along the axis 625 of their interior structure. The viral spike glycoproteins are stabilized by a multiplicity of these chloride ions 630, 635. Extracting these ions by means of FGP causes this assembly to unravel as the charge balance of this molecular structure is disrupted. Chloride ions extracted in this manner are immediately exposed to a multiplicity of hopping sodium cations bonded with the FGP 610 and may then leave the vicinity of this molecule as a sodium chloride salt (NaCl) 640. The chloride ions are sequentially ejected from the central hydrophobic region of the viral spike via van-der-Waals mediated charge disruption. They are then captured by the sodium hopping mediated nano-surfactant properties associated with the FGP. It is understood that the gallium or the sodium can be the atom which causes the charge-mediated chloride ion displacement.

The viral spike of the exemplary novel coronaviruses is composed of 6 entwined proteins of two fundamental types being the human receptor 1 (HR1) proteins indicated by the coiled coils of 645, 650, 660 and the HR2 proteins indicated by the dashed coiled coils 670, 680, 690. FGP penetrates to the hollow tubular interior of these spike viral structures to disrupt the charge symmetry and therefore the stability of these structures, causing them to eject the stabilizing chloride ions at the interior of these molecular structures. The result is to unravel the viral spike glycoproteins before they can locate a cell membrane to initiate endocytosis and become invasive to a cell of the body. This premature unravelling process is enabled by the FGP, which has a size similar to the size of the hollow central region indicated by D which is enclosed by the spike glycoproteins.

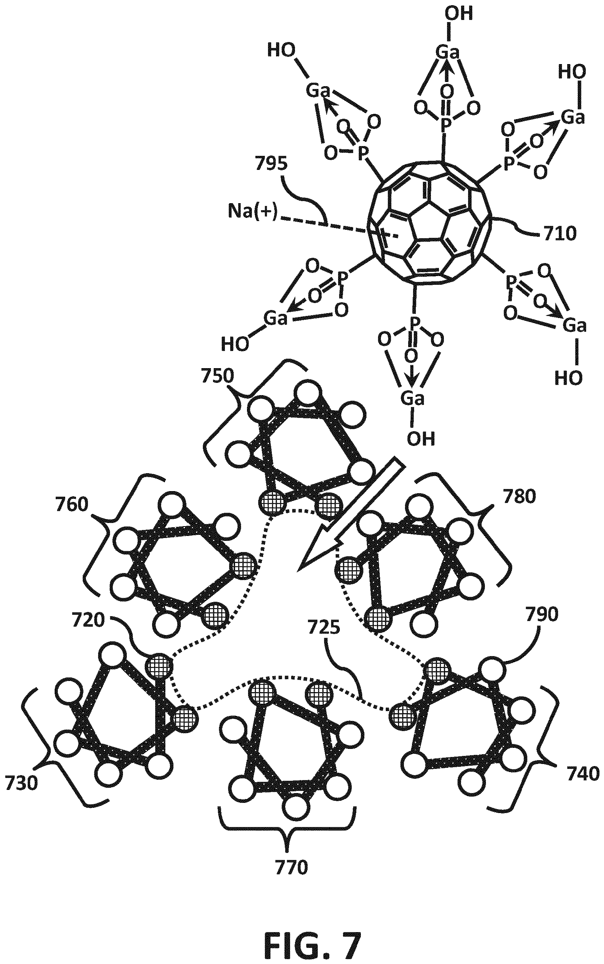

FIG. 7 is an illustration of a cross-section view the hydrophobic region of a viral spike glycoprotein from which chloride ions are to be extracted via the van-der-Waals mediated charge balance disruption illustrated in FIG. 6. The central hydrophobic hollow region of the coronavirus spike glycoprotein has an approximately triangular shape as indicated by the dotted line 730. Such structures are well documented in the x-ray crystallography literature for these viruses. The hydrophobic character of the interior region is determined by the presence of hydrophobic amino acid residues indicated by the multiplicity of circular crosshatched pattern circles 720 that abut the hydrophobic region as indicated by the dotted line 725. Viral spike glycoproteins HR1 are represented by the structures 730, 740, 750 and viral spike glycoproteins HR2 are represented by the structures 760, 770, 780 wherein each of these structures has hydrophilic amino acid residues indicated by the multiplicity of white circles 790 located at the outside or external regions of each spike glycoprotein which faces the water-soluble intercellular environment in which the virus must travel to invade the cells of the body. The large white arrow shows the direction that FGP 710 travels to enter the hydrophobic central region of the coils of the spike glycoproteins. The FGP entry process is facilitated by the presence of some pi-cation bonded sodium ions 795 carried from the physiological environment that can assist with the nano-surfactant function by means of the sodium ion hopping mechanism that was introduced for FGP in FIG. 4.

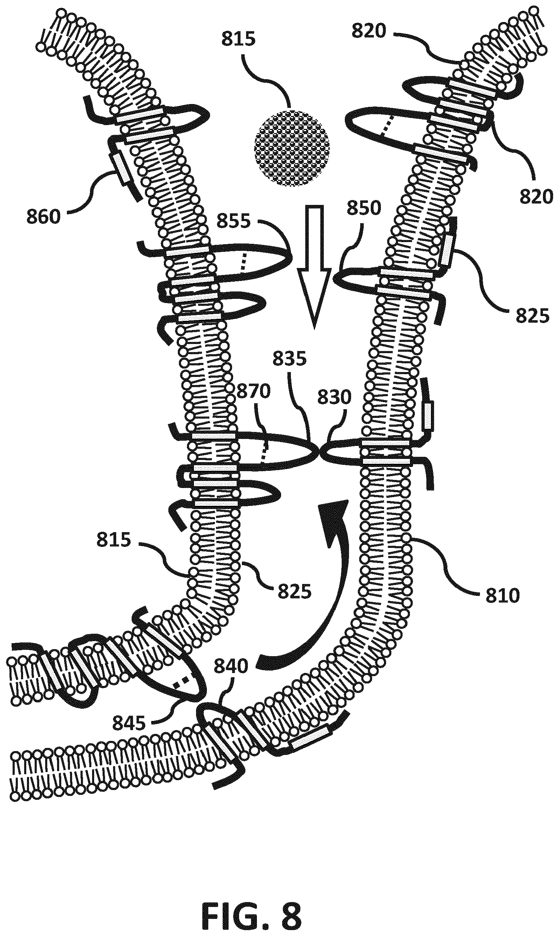

FIG. 8 is an illustration of the reconstruction and recovery of zippered cell membranes usurped and repurposed as viral replication platforms. The external phospholipid membranes of a virus infected cell organelle can be an exemplary endoplasmic reticulum of a mitochondrion 810, 815 facing the cellular cytoplasm, wherein the internal phospholipid membranes 820, 825 are facing the endoplasmic reticulum lumen. The electrostatic zipper function of an invasive pathogenic virus such as an influenza or a corona virus is enabled by large luminal viral protein loops, having oppositely induced van-der-Walls charges at the tips of the curvatures of these loops at opposing internal membrane regions. Such loops are termed 'nonstructural integral membrane proteins' or nsp; their operation is demonstrated by the coupling bonds of the abutting hydrophobic regions of high curvature in the dark black curved lines representing complementary nsp structures 830, 835, and complementary nsp structures 840, 845. A multiplicity of large, looped regions of the characteristic double loops of nsp4 structures 835, 845, 850, 855 face the interior of the mitochondrion at the lumen, each of which are stabilized by a sulfur-sulfur bridge bonding structure across the large luminal loop region represented by the dotted line 870. Nsp3 luminal loop structures 830, 840, 850, 860 provide a complementary electrostatic and hydrophobic bond forming region at the point of highest curvature of the single inward facing portion of their luminal loops. The action of nsp3 to nsp4 hydrophobic electrostatic bonding serves to bridge these opposing luminal loops of type 830 to type 835 and type 840 to type 845 to create a zippering attractive force to bring opposing inner walls of the mitochondrion into local proximity and hold them together. The narrowed gap region between the now proximal phospholipid membranes serves as a platform to assemble the replicating virus. The zippering direction of this movement is shown by the large curved black arrow. FGP 815 is provided to counteract the loop zippering function, by inserting itself into the regions between abutting nsp3 850 and nsp4 855, as indicated by the pointing direction of the large white arrow. This allows electrostatic charges to be introduced to at least one hydrophobic portion of 850 and 855 by the induced bonding of a hydrophobic portion of the FGP 815 as a decoration onto the high curvature portion the loop which acts to prevent future zippering effects by means of its hydrophilic gallium hydroxide functional groups and any pi-cation bonded sodium ions.

The region of the white arrow denotes an electrostatic bilayer in transition away from the zippered region of electrochemical charge storage between opposingly charged van-der-Walls hydrophobic faradic viral protein loops in the nanoconfined spaces of the region of the black arrow. FGP 815 enables the separation and restoration of cell membranes by being neither a purely electrostatic material nor a purely faradaic material. It should rather be regarded as a catalyst that enables a continuous unzippering between the abutting internal phospholipid membranes. The hopping of the sodium ions is a reversible process in which the exchange of locations takes place from a somewhat non-polarizable (faradaic) region at the phosphonate to a somewhat polarizable (non-faradaic) region at the fullerene carbon face over a hopping distance of less than five nanometers. The unzippering process constitutes a subtle chemical mechanism that has now been harnessed to reverse a pathological biochemical condition operating on infected cellular components as propagated by an invasive virus.

It is notable that the physiological pH within the mitochondrion will cause negative ionic faradic charges to appear at the terminal ends of at least some of the pendant phosphonate groups. Faradic charge is the expression of continuous electrostatic interactions that exist between charged or polar surfaces and extend into water which is a polar molecule. However, transient, or induced charges appear at the fullerene group where the carbon faces provide induced van der Waals forces to create a transient opposing charge in an uncharged or non-polar hydrophobic abutting surface, such as provided by the ability to adhere to a first nonpolar nsp viral protein. Those portions of the molecular structure provided with charged fullerene phosphonates being of hydrophilic or polar nature are then able to repel the hydrophobic region at the point of maximum curvature of any second abutting or nearly abutting nsp loop. The fullerene phosphonate thereby acts as an electrostatic cap to prevent the induction of opposing charges with any zippering nsp luminal loop. This unzippering function of the fullerene phosphonates is designed to disable the nsp from finding and recruiting any partner nsp loop for the purpose of establishing the hydrophobic zipper of the platform required to replicate virus particles. Paired nsp types of viral protein structures similar to those released by the coronavirus nsp3 and nsp4 glycoproteins have been identified in hepatitis virus, especially that of hepatitis B or (HBV). It is therefore a purpose of the present invention to halt the recruitment of partnering nsp of any type, from any virus particle proteins expressing a van der Waals paired nsp membrane zippering function. The electrostatic or faradic capping function of FGP halts the virus from creating replication platforms within cellular mitochondria or other cellular organelles when the FGP promotes conditions unfavorable to viral replication, according to these teachings.

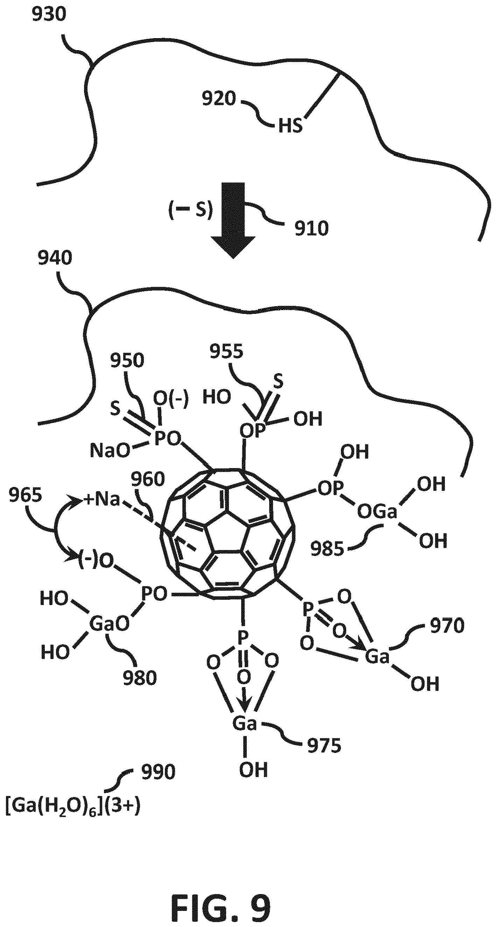

FIG. 9 is an illustration of the protease desulfurization reaction with FGP wherein the phosphonate groups extract catalytic sulfur from within the protected cavity of the viral or fungal protease. The desulfurization reaction proceeds in the direction of the large black arrow 910 indicating (-S) for the sulfur extraction from the pendant sulfhydryl group 920 at a cysteine or other sulfur containing amino acid. The size of the fullerene sodium phosphonate must be sufficiently small to enable entry into the protected protease catalytic fold or cavity within the overall protease structure 930. The extraction of sulfur from the protease 940 leaves this protease unable to digest the proteins of the human body and thus renders it unable to provide raw materials in the form of amino acids to convey to the invasive fungus or viral replication platform. At least one abutting phosphorous atom reacts to form sulphurated phosphates 950, 955. Each sulphurated phosphate group obtains a phosphorus atom at an oxidation state of 5 in this example of the desulfurization reaction. Physiological sodium cations are attracted to the FGP molecule to form reversible pi-cation bonds with the aromatic regions of the fullerene 960, which then undergo charge hopping 965 with a negatively charged oxygen group at a phosphonate, thereby providing a nano-surfactant function to enable the deep penetration of yet other protease structures such as 930, 940 to enable the subsequent penetration of the unreacted sodium phosphonates in an oxidation state of 3. The FGP contains gallium hydroxide functional groups 975, 980 that continue to hydrolyze away from their respective phosphonates, such as at 980, 985 that leads to more exposure of the phosphonates to sodium cation exchange, with the eventual release of gallium as the gallium ion 990. Desulfurization by FGP assists the human body to penetrate and deactivate destructive viral and fungal proteases, such as the main protease 3 (MPRO3) associated with the SARS-Cov-2 coronavirus strains, while the released gallium ion 990 is attracted to sites of inflammation as a therapeutic antimicrobial, according to these teachings.

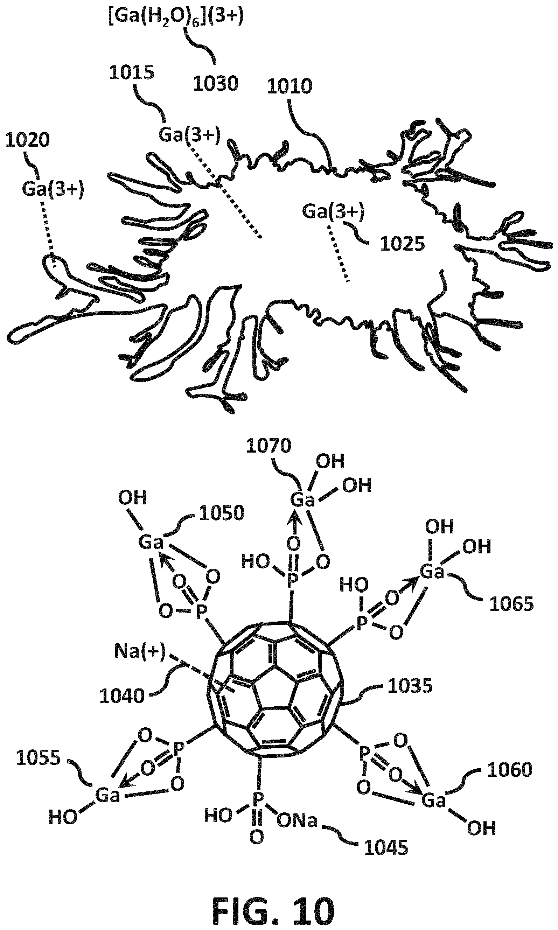

FIG. 10 is an illustration of the gallium donor function of FGP to hypoxic cancer cells. The proliferative neoplasm 1010 is a characteristic cell within tumors and cancers. It is medically well-understood that solvated gallium ions 1015, 1020, 1025 are attracted to and bond with neoplasm 1010 enabling their apoptosis or programmed cell death. Gallium ions are transported in highly hydrated form 1030 and will be carried by the blood plasma along with FGP 1035 which functions to release gallium ions through their hydration. Incursion of the FGP to neoplasms is expedited by the presence of ambient physiological sodium ions which can form pi-cation bonds 1040 with the fullerene group that act as a nano-surfactant while reversibly hopping to negatively charged phosphonate oxygen atoms to form the NaO functional group 1045.

An alternative way to kill living tumor and cancer cells that can be supplemental to any combination of cancer vaccines, chemotherapy, and radiation treatments, is the use of Ga3+ ions to substantially remove the ability of these kinds of rapidly proliferating cells to respire using the glycolysis cycle of respiration. Glycolysis cellular respiration strongly depends on the presence of iron as Fe3+ in the environment and is reminiscent of the function of single cell bacterial life in hypoxic (low oxygen) environments. Iron forms bonds with oxygen and creates the free radicals (reactive oxygen species or ROS) that are needed by the REDOX reaction to process glucose in the glycolysis respiration cycle of cancer and tumor cells. Healthy cells utilize the oxidative phosphorylation (OXPHOS) cycle which is much more robust than the glycolysis cycle. Ga3+ is an iron mimetic that is not a redox-active ion. Gallium cannot make a free radical. The proliferative cells will mistakenly accept Ga3+ in place of Fe3+ in their environment to a far greater detriment to their respiration than will be the case for healthy cells having an alternative OXPHOS pathway to respiration. This is one way that Ga3+ kills cancer cells and old or malfunctioning (senescent) cells by interfering primarily with glycolysis respiration, while leaving healthy cells substantially unaffected. In another mechanism, Fe3+ is required for DNA replication. For fast replicating proliferative cells, the dilution of some Fe3+ by Ga3+ can terminate the replication process. For slowly replicating healthy cells, Ga3+ will soon enough be displaced again by Fe3+ so that the temporary slowdown in replication is not usually significant.

Cancer cells create within themselves a hypoxic or low-oxygen environment, which is significantly attractive to the hydroxylated gallium phosphonate groups 1050, 1055, 1060, as well as to those gallium groups having more hydrolysis and functional hydroxylations 1065, 1070. By bonding ionic gallium to neoplasms in place of iron, the neoplasms fail to survive using a glycolysis type of respiration, as this requires a high concentration of iron cations as Fe2+ and Fe3+ (not shown). Because neoplasms can no longer switch to the oxidative phosphorylation cycle, and gallium ions have replaced many of the sites of active iron required to proliferate cancerous DNA, these cells and related tumor cells will die. Ordinary healthy cells will survive and flourish even in the presence of gallium, because they are able to use oxidative phosphorylation to maintain a good supply of oxygen to generate cellular environments that are not significantly attractive to gallium ions, according to these teachings.

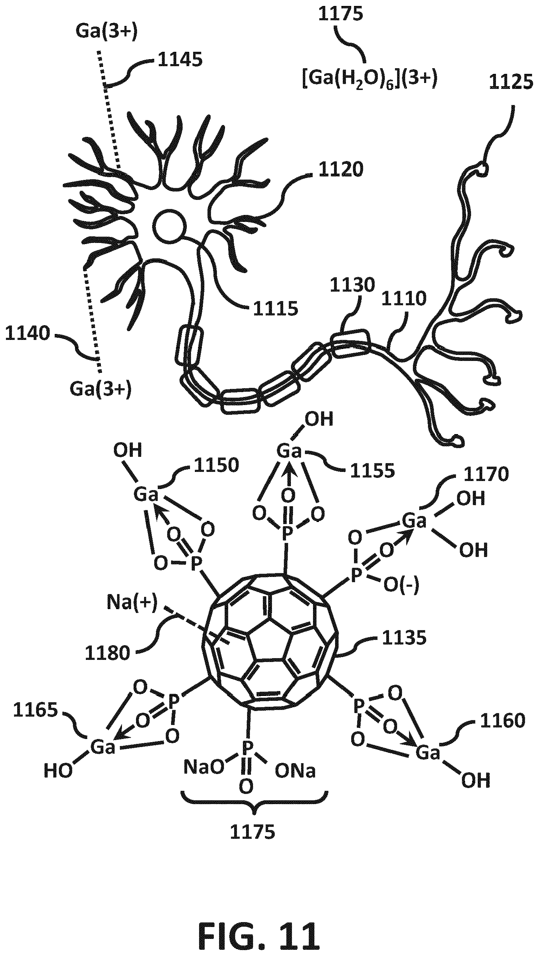

FIG. 11 is an illustration of the neurite growth function of FGP to repair and remediate neurological deficits such as Duchenne's muscular dystrophy. The neuron 1110 is a characteristic cell within both the body, it actuates muscle contractions in the body, and has a functional role in the brain. The neuron 1110 has a cell nucleus 1115, dendrites 1120 to interface with other cells, a multiplicity of synaptic boutons 1125 to accept electrochemical and electrical signals from other neurons, and a multiplicity of fatty myelin sheaths 1130 that insulate neural signals to prevent signal loss during transmission. Genetic defects can combine with environmental stressors such viral infections to create within some neurons 1110 an inability to properly heal themselves and grow new neurites 1120, 1125 to maintain proper communication and interfaces with other cells. FGP molecule 1135 functions to donate gallium ions having a valence of three as Ga3+ 1140, 1145 to initiate new neurite growth at dendrites 1120. The donated gallium ions having a valence of 3 are a hydrolysis product that can arrive at the neuron 1110 in the form of six ligands with water. Hydrolysis of any of the exemplary gallium groups of FGP 1150, 1155, 1160, 1165 proceeds by accepting one hydroxyl group from ambient water to form a gallium functional group with two pendant hydroxides 1170. An additional hydrolysis results in the final third hydroxylation of the gallium ion to release it from the phosphonate and allow it to form the aqueous gallium 1175, thereby leaving behind a phosphonate group that can ion exchange with the ambient physiological sodium ions to form pi-cation bonds as indicated by the dashed line 1180. The sodium ions can then reversibly charge hop to the phosphonate groups within 5 nanometers of the fullerene group of FGP 1135 to provide the nano-surfactant function which allows the FGP molecule and pendant gallium functional groups to penetrate the neural dendrites 1120, where they collectively act as nucleation agents to stimulate new dendritic growth. The generation of new dendrites has multiple beneficial and therapeutic effects, as it can alleviate conditions such as Duchenne's muscular dystrophy as well as other dystrophies by helping to re-establish communication with interfacing cells, and by helping to remediate sites of damage from activities or infection as the predominant sources of environmental stress. The gallium cations released by fullerene gallium phosphonate also act as an antimicrobial agent to protect the fine structures of neurons such as dendrites 1120, and synaptic boutons 1125 from disruptive attack by invasive fungi and bacteria, as well as to inactivate virus particles. It is already well-known that gallium cations induce neural stem cells and neural precursor cells to differentiate into neurons and astrocytes. In the more developed neurons, the cations of Ga3+ become charge attracted to the negatively charged neural boutons in the same natural manner as would any other cations such as iron cations. Unlike iron cations, Ga3+ attraction does not generate damaging free radicals or cause neural inflammation as with the iron cations Fe2+ and Fe3+, thereby guaranteeing no neural damage through such stimulation. Ga3+ can then interact with basic fibroblast growth factors that are already present at the neural boutons to promote additional neurite growth without damage to these boutons, according to these teachings.

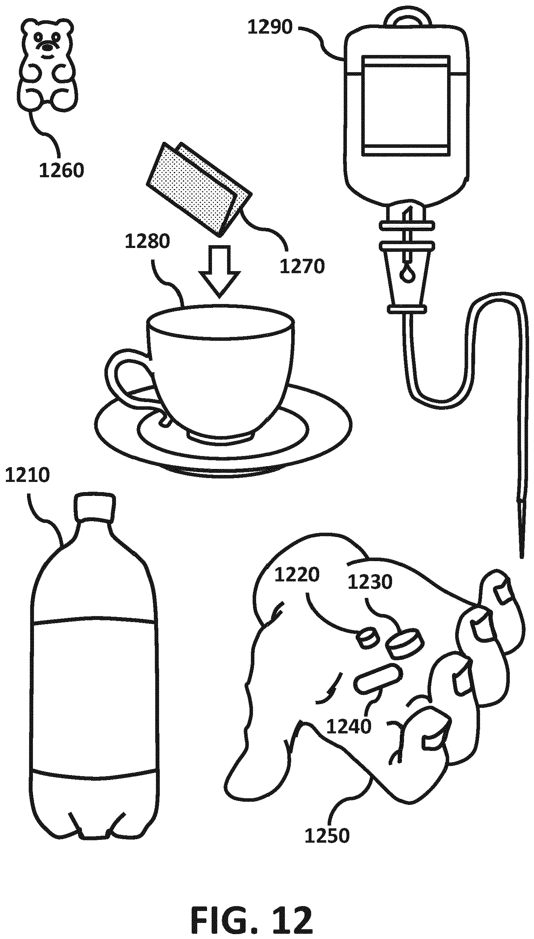

FIG. 12 is an illustration of exemplary methods to administer formulations to contain and deliver fullerene gallium phosphonates. In these non-limiting examples, the FGP is dissolved into a beverage such as a carbonated soft drink 1210 or an alcoholic drink. It is also understood that other types of beverages such as wine, champagne, beer, and fruit juices are amenable to the addition of FGP. Another method of delivery or dosage of FGP is by pill 1220, tablet 1230 or powder-filled hard gelatin capsule 1240 which are portable and easily administered by hand 1250. Dilution of FGP into edible powders includes baking powder (sodium carbonate), and baking soda (sodium bicarbonate) in any combination. In an alternative delivery method, the FGP can be added to a gelatin gummi having a soft pliable dry form that may be shaped as desired, such as in the form of a 'gummi bear' 1260. In yet another method of delivery or serving, the FGP powder may be added to a sugar or a sugar substitute and packaged into individual sweetening packets 1270. Such packets can be opened and added to a liquid brewed beverage such as a tea or a coffee 1280. Another method of delivering FGP is by intravenous infusion therapy (IV drip) 1290, in which the FGP molecules are attached by faradic attraction to the biomolecules of human blood plasma for subcutaneous application; an infusion pump can be used to adjust the delivery rate through a catheter that is to be inserted into the localized area to be treated.

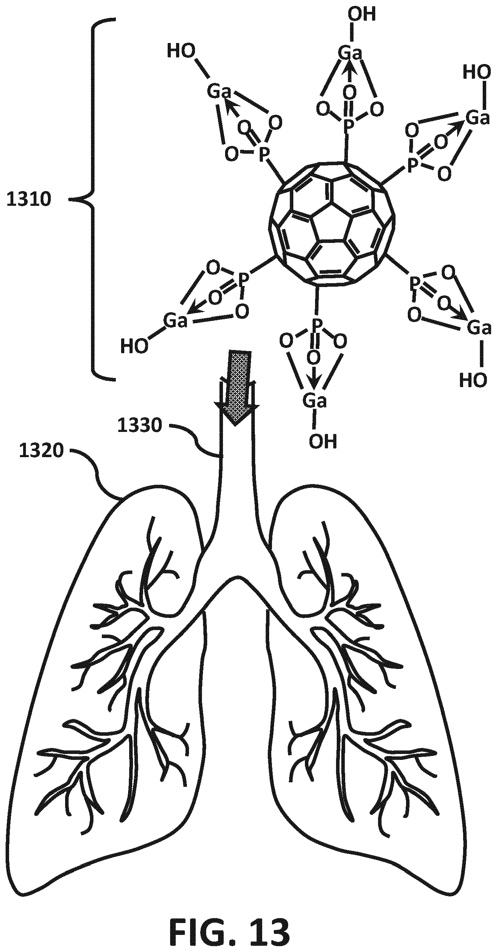

FIG. 13 is an illustration of fullerene gallium phosphonate aspirated into the lungs for preventative or therapeutic treatment of any microbial-induced respiratory ailment that can benefit from this method of breath mediated delivery. FGP 1310 are aspirated into the lungs 1320 and airways 1330 to deactivate a viral pneumonia or other invasive pathogen such as a fungal spore infection causing a cytokine storm followed by the lungs filling with mucus composed of pulmonary surfactant (pus). FGP, being heavily negatively charged, is attracted to and binds with endogenously produced pulmonary surfactant in the airways. The surfactant properties of FGP in the presence of physiological sodium ions allows the charge-hopping nano-surfactant mechanism of this molecule to act to significantly reduce surface free energy, increase wetting ability, and lessen the viscosity of the biological mucus so that these biomaterials can be expeditiously returned as part of the blood plasma to the vascular circulatory system. The effect of the FGP is to clear the lungs and airways of the patient so that breathing is no longer obstructed, and an exchange of oxygen and carbon dioxide gases allows normal and healthy respiration to be established. The antifungal properties of FGP assist with the treatment of valley fever, as well as other respiratory illnesses such as pneumonia, chronic obstructive pulmonary disorder (COPD), and some types of bacterial infections of the lung such as antibody-resistant bronchitis and tuberculosis by directly treating the affected lung tissues, according to these teachings.

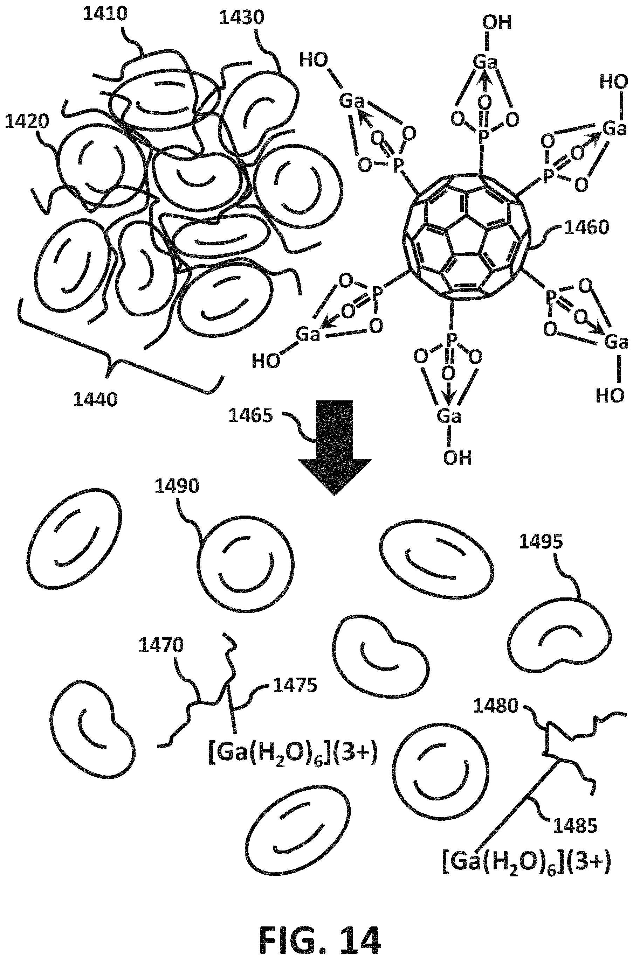

FIG. 14 illustrates the effect of fullerene gallium phosphonate to remediate fibrin mediated blood clots and treat hypertension. Iron ions Fe2+ and Fe3+ as well as their hydroxides in blood plasma alter fibrin molecules by accelerating the polymerization of fibrin monomers to form a multiplicity of entangled fibrin polymers 1410 which cause a multiplicity of blood cells 1420, 1430 to become entangled in a blood clot 1440. The donation of gallium as Ga2+ or Ga3+ and their hydroxides from at least one molecule of fullerene gallium phosphonate 1460 serves to mask those sites that may otherwise bond with iron ions, thereby causing a reaction as shown by the direction of the large black arrow 1465 pointing to free fibrin strands 1470, 1480 bonded temporarily with gallium hydroxides 1475, 1485 and providing a free path for the multiplicity of blood cells 1490, 1495 to escape into the blood plasma of the vasculature. The anti-inflammatory properties of fullerene gallium phosphonate and gallium ions as well as that of gallium hydroxides reduce the recruitment of cytokines and chemokines.

Insoluble fibrinogen polymer forms by the action of hydroxyl free radicals caused by iron cations. However, gallium cations are unable to initiate a free radical on hydroxyl groups. Fullerene functional groups are well known to be able to quench free radicals. The combined effects of both removing free radicals by fullerene and never allowing them to form initially by the presence of sufficient gallium 1475, 1485 creates a highly synergistic biological free radical quenching mechanism. This enables the rapid recovery of fibrin monomers from their polymeric and blood clotted forms. Removal of fibrin polymers from the walls of the vasculature is furthermore assisted by the solvation of oxidized fats enabled by the nano-surfactant properties of fullerene gallium phosphonates to significantly enhance and restore the native plasticity of hardened veins and arteries. The nano-surfactant properties of fullerene gallium phosphonate also serve to keep the fibrin and red blood cells solvated with the water phase of the blood plasma, and apart from each other, by means of the charge screening of oriented water which forms around the positively charged gallium ions and the fullerene gallium phosphonates, according to these teachings.

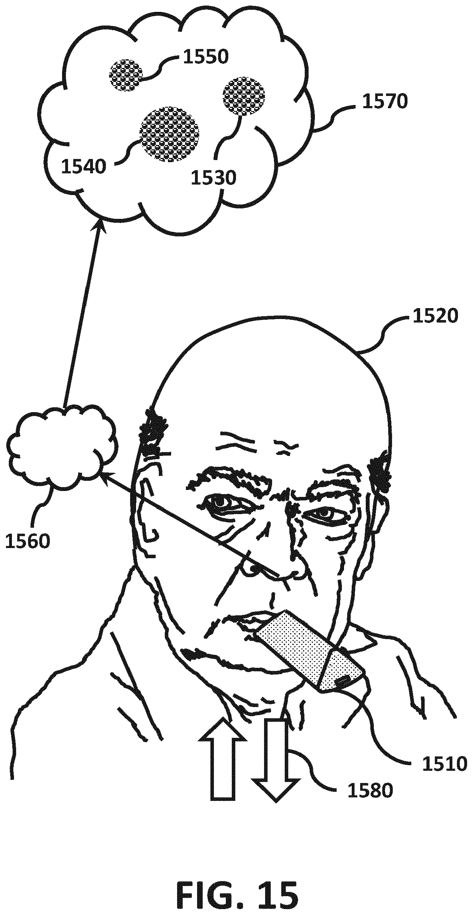

FIG. 15 is an illustration of the personal administration of an aspirated nano-aerosol delivery solution containing the fullerene gallium phosphonate molecules of the present invention. A nano-aerosol generating device 1510 filled with the fluid mixture containing the FGP molecules of the present invention as an inhalant dispensing solution that is provided for dispersing the created inhalant gas wherein the FGP nanoparticles are nebulized. The dispensing device 1510 may also be more commonly known as a nebulizer, or an electronic vaporizing device, or an electronic cigarette, or the functional part of a hookah to be shared among several users. In all cases these systems serve to carry the composition in a carrier fluid dispenser 1510, move that composition in a nebulized form along with an aerosolized solvent, and transfer this composition into a substantially gaseous dispersion directed into the nose, mouth, trachea, and airways of a patient or user 1520. One intended use of the composition is to treat, delay or arrest the incidence of brain cancers wherein the nano-aerosol can expedite targeted delivery to the brain by avoiding a passage through the digestive system. Another intended use is to treat COPD, pneumonia, and other respiratory ailments using the composition of the present invention. A volume of an inhalant in the form of an air dispersed nano aerosol, a water mist dispersed vapor, an air dispersed powder, an air dispersed dust, or an air dispersed aerosol can be administered, in various embodiments. An exemplary daily dosing comprises an aspirated volume of 400 ml that is inhaled for 3 seconds for a cumulative dose of 0.0015 mg to 0.0020 mg of FGP delivered to the lungs and airway passages, dispensed from a solution or dust containing 500 parts per million FGP.

Some of the nano-aerosolized composition is exhaled and shown as particulate clusters 1530, 1540, 1550 within exhaled smoke puffs 1560 and 1570 emitted on exhalation as indicated by the direction of thin line arrows pointing away from the nose of the subject 1520. Delivery of the nano-aerosol FGP composition from dispenser 1510 provides antimicrobial properties to the mucus airway tissues wherein destruction of microbes associated with viral infection, fungal infection such as valley fever, or to treat COPD can be provided using this method. Systems that may be used for the method of dispersion of the nano-aerosol fluid is represented by dispenser 1510, and include, without limitation, any of the electronic cigarette devices produced internationally and listed in Appendix 4.1, "Major E-cigarette Manufacturers" of the "2016 Surgeon General's Report: E-Cigarette Use Among Youth and Young Adults" published by the Center for Disease Control and Prevention (CDC), Office of Smoking and Health (OSH) available at the CDC.GOV website, and / or any combination of piezoelectric, resistively heated, or inductively heated vaporized fluid delivery methods that can be utilized to deliver the composition of the present invention, especially when such a device is approved as a medical drug delivery device. Each embodied variation of such methods without limit are intended to aspirate aerosols as the method of therapeutic substance delivery of the FGP composition of the present invention directed into the nasal cavities, mouth, tracheal breathing orifice, or intubated trachea of a patient. The supply direction of nebulized feed on inhalation and exhalation are delivered into the airways and lungs of the intended patient by the flow of supplied air as indicated by the direction of upward and downward facing large white arrows 1580, when used according to these teachings.

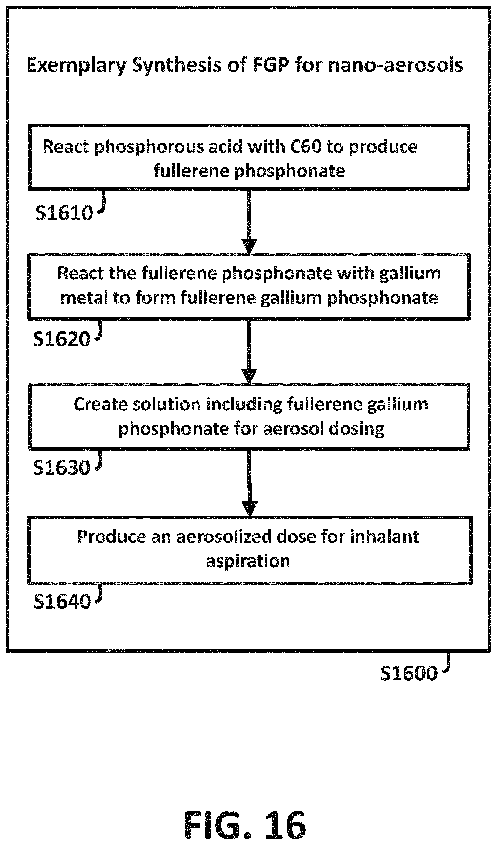

FIG. 16 is a flowchart representation of an exemplary scalable method S1600 for the synthesis of fullerene gallium phosphonate, the further production of a nano-aerosol formulated for inhalant administration of the fullerene gallium phosphonate, and the creation of an aerosol therefrom. In step S1610 one mole of buckminsterfullerene (C60) is reacted with a stoichiometric amount of phosphorous acid. The C60 can be vacuum purified, for example, while the phosphorous acid can be dry crystalline phosphorous acid, in various embodiments. It is noted that the stoichiometry can be adjusted based on the desired number of poly-gallium hydroxylations for the synthesized phosphonate desired for making the FGP.

In some embodiments of step S1610, the C60 and phosphorous acid are first mixed together to form a dry powder mixture that is then reaction shear milled. In an exemplary reaction shear milling process, the temperature range is from about 50°C to about 95°C and the shear rate is about 1000 per minute to achieve the desired intermediate product fullerene phosphonate. In this process, a shear pressure of about 20 grams per square micron is sufficient to create a slightly geometric oblate spheroid of the C60 functional group to shift the density of states of the electrons of the carbon cage molecule into transient anisotropic electrostatic charge distributions suitable for reaction. Alternatively, the standard microwave irradiation of 2.45 GHz can induce the required interaction with water or phosphonate groups having electrostatic dipoles to help enable a faster reaction rate with no other differences expected for this reaction to proceed.

In step S 1620, gallium metal is added, and the reactive shear mixing is resumed under the same conditions for another 25 minutes. In some embodiments 0.5% by weight of gallium metal is added to the fullerene phosphonate product of the prior step. The product of step S1620 is a powder of fullerene gallium phosphonate suitable for the various purposes outlined herein.

In step S1630, a solution is created with which an aerosol containing fullerene gallium phosphonate can be made. In one embodiment, an amount of the dry FGP powder from step S1620 is dissolved into a solvent mixture such as 70% glycerol and 30% polypropylene glycol by volume. The amount added is sufficient such that an aerosolized dose thereof will deliver a pharmaceutically effective amount of the fullerene gallium phosphonate.

In step S 1640, an aerosolized dose for inhalant aspiration is produced, for example, by metering an amount of the nano aerosol fluid from step S 1640, where the amount includes a pharmaceutically effective dose of fullerene gallium phosphonate. For instance, this step can be performed by a commercially available electronic dispensing device suitable for client inhalant aspiration by means of a heated airflow between about 255°C and 300°C to create the gaseous nano-aerosol, according to these teachings. the further production of a nano-aerosol formulated for inhalant administration of the fullerene gallium phosphonate, and the creation of an aerosol therefrom.

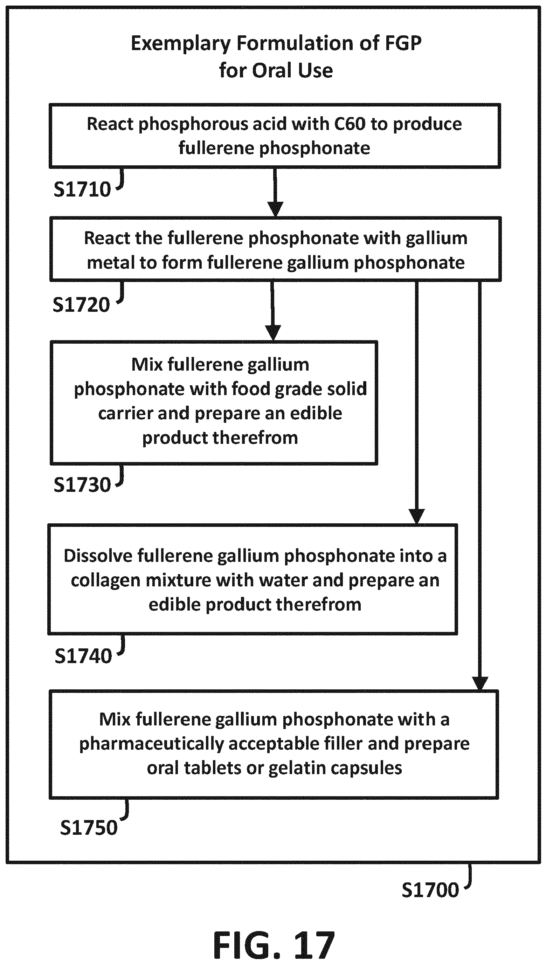

FIG. 17 is a flowchart representation of an exemplary scalable method S1700 for the synthesis of fullerene gallium phosphonate, and the further production of formulations of fullerene gallium phosphonates (FGP) for various oral administrations. Steps S1710 and S1720 are the same as steps S1610 and S1620 of FIG. 16.

In optional step S1730 a quantity of fullerene gallium phosphonate from step S1720 is mixed into a food grade solid carrier such as a zeolite, baking powder (sodium carbonate), calcium carbonate, baking soda (sodium bicarbonate), collagen powder, natural or artificial sweetener, or similar powdered solid phase to produce an edible product. For example, an exemplary 1-kilogram loaf of antimicrobial bread can contain 10 grams of baking soda or baking powder fortified with 500 mg of FGP to yield 50 mg of FGP per slice of bread at 10 equal slices per loaf, such that a daily administration of two slices provides 100 mg FGP per day. In optional step S1740 a quantity of fullerene gallium phosphonate from step S1720 is added to a collagen mixture with water, optionally with one or more of food grade colors, flavors, and potassium sorbate, preservative to produce a flexible gelatin gummi for oral administration.

In optional step S1750 a quantity of fullerene gallium phosphonate from step S1720 is mixed with a pharmaceutically acceptable filler and compacted to produce an oral tablet or added into hard gelatin powder capsules for exact dosages. It is understood that other methods for oral consumption and administration or variations of these methods can be found satisfactory and able to convey an amount of FGP to the human or animal patient.

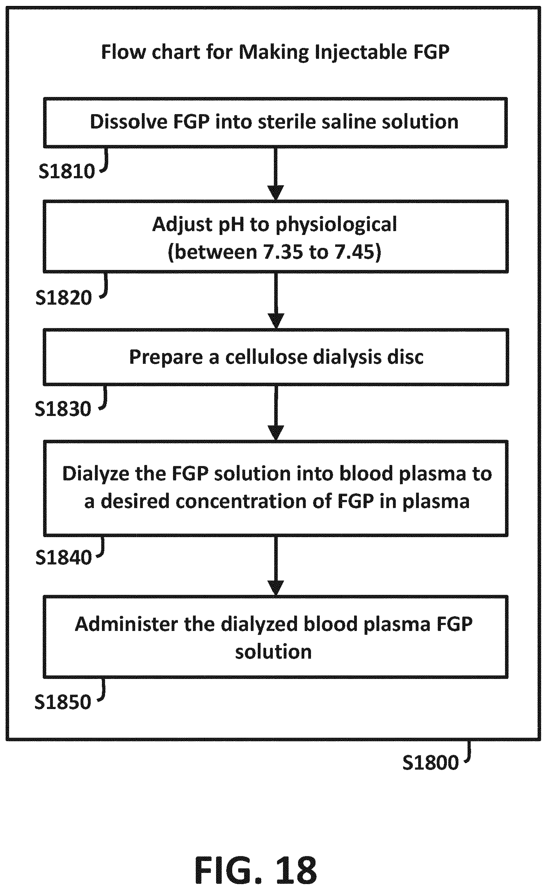

FIG. 18 is a flowchart showing exemplary steps of a method S1800 to prepare and administer fullerene gallium phosphonate in blood plasma intravenously. In step S1810, fullerene gallium phosphonate is dissolved into sterile distilled water. In some embodiments ultrasound and/or mechanical stirring is employed until the solid particulates visibly disappear. The fullerene gallium phosphonate can be produced, for example, by the method outlined in steps 1610 and 1620 of FIG. 16. If necessary, in step S1820 the pH of the fullerene gallium phosphonate solution from step S1810 is adjusted to a physiological pH from between about 7.35 to about 7.45.

In step S 1830, a cellulose dialysis disc having a molecular weight cutoff of 3500 Daltons, for example, is rinsed with distilled water to remove any packaging glycerol. An exemplary 33 mm disc is the Spectra/Por^{™} RC, manufacturer number 132488, provided by Repligen at 18617 South Broadwick Street, Rancho Dominguez, California 90220, USA. In step S 1840, an aqueous FGP solution is dialyzed, in a cellulose dialysis bag for example, into blood plasma to obtain the desired concentration of FGP in sterile blood plasma at a physiological pH. By passing through the dialysis filter, potential impurities greater than 3500 Daltons as well as possible dust is removed.

In step S1850, the sterile dialyzed blood plasma FGP solution is administered by injection or intravenous (IV) drip to the patient as specified by an attending physician. A drip rate may be adjusted by an infusion pump to vary depending on the prescribed dosage and concentration, which can relate to the body weight of the patient as well as the severity of the microbial load to be addressed, which has been determined in the patient.

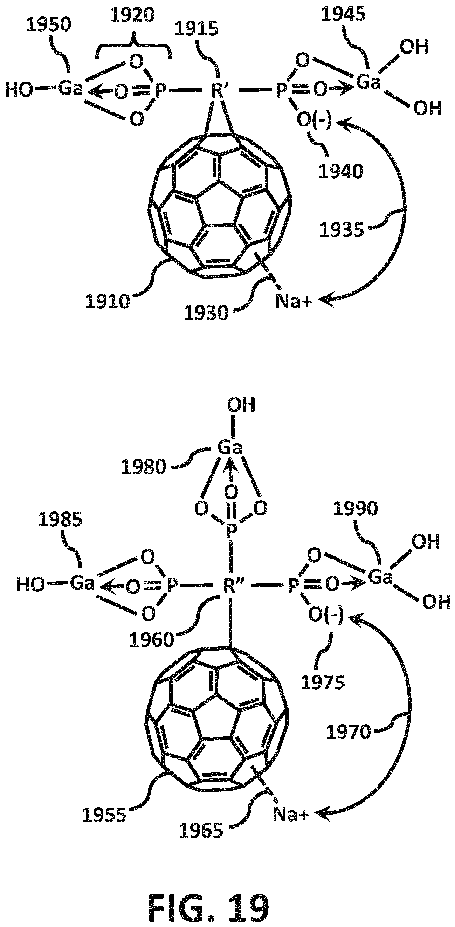

FIG. 19 is an illustration of alternative fullerene molecular structures with intermediate organic functional groups that contain FGP. Bisphosphonate 1910 has an organic functional group R' 1915 containing at least one carbon that is bonded to two adjacent fullerene carbons. The R' group 1915 is also bonded to two phosphonate groups, where one of these is indicated by the bracketed region 1920. More than one R' group 1915 with bisphosphonates may be bonded to the same fullerene molecule. At least one ambient physiological sodium ion becomes pi-cation bonded with the aromatic regions of the fullerene group as indicated by the dashed line 1930 to enable it to hop reversibly as indicated by 1935 to a phosphonate negative charged oxygen atom 1940 which is less than 5 nanometers away in this molecular structure. Gallium atoms of valence three are bonded to at least one of the phosphonate oxygens with a lone pair of electrons from a double bonded oxygen atom contributing to the stability of this functional group, while two hydroxyl groups can then also bond to the gallium atom, 1945. However, gallium 1950 can simultaneously bond covalently with two atoms of a phosphonate group while also withdrawing electronegativity from the double bonded phosphonyl oxygen and have one covalent bond to a hydroxyl group. The gallium hydroxyl groups serve to anchor the FGP onto the hydrophilic outside of the viral protein structure, while the hydrophobic and net negative charged fullerene face destabilizes and disassembles the charge stabilized viral protein structure such as a spike glycoprotein or a capsid on the forced exit of its charge-stabilizing chloride ion. The overall size of the fullerene gallium bisphosphonate 1910 is sufficiently small and labile to allow insertion of at least one hopping pi-bonded sodium cation at a hydrophobic carbon face to combine with a chloride pinning ion holding together a virus protein structure. The chloride ion then breaks away from the central cavity formed by the nested glycoprotein molecules, taking with it the negatively charged electron which used to be the bond between the chloride ion and the positive charged amine groups at that location. The chloride ion then attracts to the positive charged sodium ion, which leaves to form NaCl, or to allow the insertion of a gallium cation as a gallium hydroxide to perform the same function.

Fullerene tris-phosphonate 1955 has an organic functional group R" 1960 containing at least one carbon that is bonded to one adjacent fullerene carbon atom. The R" group 1960 is bonded to three phosphonate groups. More than one R" group 1960 with tris-phosphonates may be bonded to the same fullerene molecule. At least one sodium ion is pi-cation bonded with the aromatic regions of the fullerene group as indicated by the dashed line 1965 to enable it to hop reversibly as indicated by 1970 to a phosphonate negative charged oxygen atom 1975 which is less than 5 nanometers away in this molecular structure. Gallium ions of valence three are bonded to at least one of the phosphonate oxygens with a lone pair of electrons from a double bonded oxygen atom contributing to the stability of this functional group 1980, 1985 while two hydroxyl groups can also bond to the gallium atom 1990. In each case of gallium bonded to a phosphonate, gallium simultaneously withdraws electronegativity from the double bonded phosphonyl oxygen as indicated by the small arrow pointing from it to the gallium atom. Any of the gallium hydroxyl groups serve to anchor the FGP onto the hydrophilic outside of the viral protein structure, while the hydrophobic and net negative charged fullerene face destabilizes and disassembles the charge stabilized viral protein structure such as a spike glycoprotein or a capsid to enable the forced exit of its charge-stabilizing chloride ion. The overall size of fullerene gallium tris-phosphonate 1955 is sufficiently small and labile to allow insertion of at least one hopping pi-bonded sodium cation at a hydrophobic carbon face to combine with a chloride pinning ion holding together a virus protein structure to remove it by the formation of NaCl.

It is generally understood that the product of three simultaneous addition reactions onto the same fullerene molecule results in a tris-fullerene product. Therefore, a single organic molecule adding to two carbon atoms of one fullerene is considered a bis-fullerene as for R' 1915, whereas two of organic molecules of R" 1955 adding to two carbon atoms of one fullerene is also considered a bis-fullerene. Three simultaneous addition reactions of either R' or R" to one fullerene molecule are generally considered tris-fullerenes even though three of the R' may react with six carbon atoms of the same fullerene molecule. In all cases, there must be at least one phosphonate group on at least one reactant adduct to allow the subsequent reaction with gallium to create a bis-FGP or a tris-FGP.

The antimicrobial properties of bis-phosphonate gallium fullerenes and tris-phosphonate gallium fullerenes, being partly saponified on exposure to sodium cations under physiological conditions and having an intermediate organic group that is bonded to the fullerene, can be acceptable antimicrobial alternatives to fullerene gallium phosphonates having phosphorus directly bonded to the fullerene. It is to be understood that the pharmacokinetics and efficacious dosage can be different in such alternative structures, wherein it is generally known that greater mass molecular structures will diffuse more slowly than lighter molecular structures, in accordance with the teachings of the present invention.

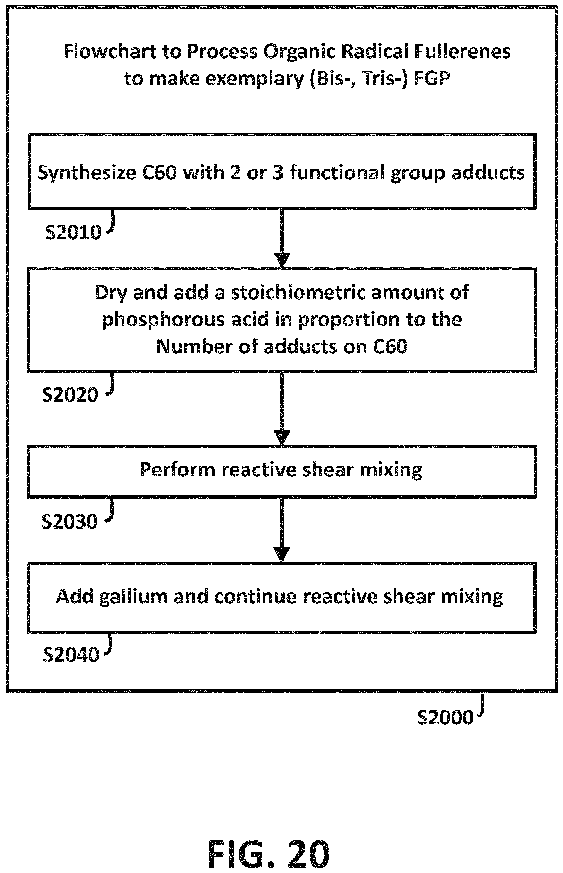

FIG. 20 is a flowchart showing exemplary steps to prepare fullerene gallium phosphonate having organic radical groups between the fullerene and the gallium phosphonate. It is understood that a wide variety of purified bis-fullerenes and tris-fullerenes bonded to carbon containing functional groups are commercially available. However, it is also possible to synthesize these. In step S2010 one of several well-known synthesis reactions are employed to produce one or more simultaneous carbon additions onto a C60 molecule. For instance, the wet chemical condensation of a desired R-amino acid with an aldehyde and a C60 is performed in a solvent matrix such as toluene in accordance with the well-known Prado reaction. The solvent should be completely removed after this reaction is completed. Step S2010 may also be accomplished using, for example, the well-known Bingel reaction for C60. This step is not limited to 2 or 3 functional group adducts and can include more.

In step S2020, the bis- or tris- or multiply substituted organic radical fullerene is dried and a mixture is made by adding a stoichiometric amount of phosphorous acid in proportion to the organic radical adducts on the C60 with which these are to react.

In step S2030 reactive shear mixing at about 1000/sec at about 55°C for about 25 minutes is performed while applying about 20 grams per square micron shearing pressure to form the phosphonate groups at the organic radicals bonded to the C60.

In step S2040 a stoichiometric amount of gallium is added to substantially neutralize the phosphonate acid hydrogen groups with gallium cations and the reactive shear mixing process is resumed, thereby forming the type of fullerene gallium phosphonate that is provided with an intermediate linkage through an organic functional group as a bis type R' or as a tris type R".

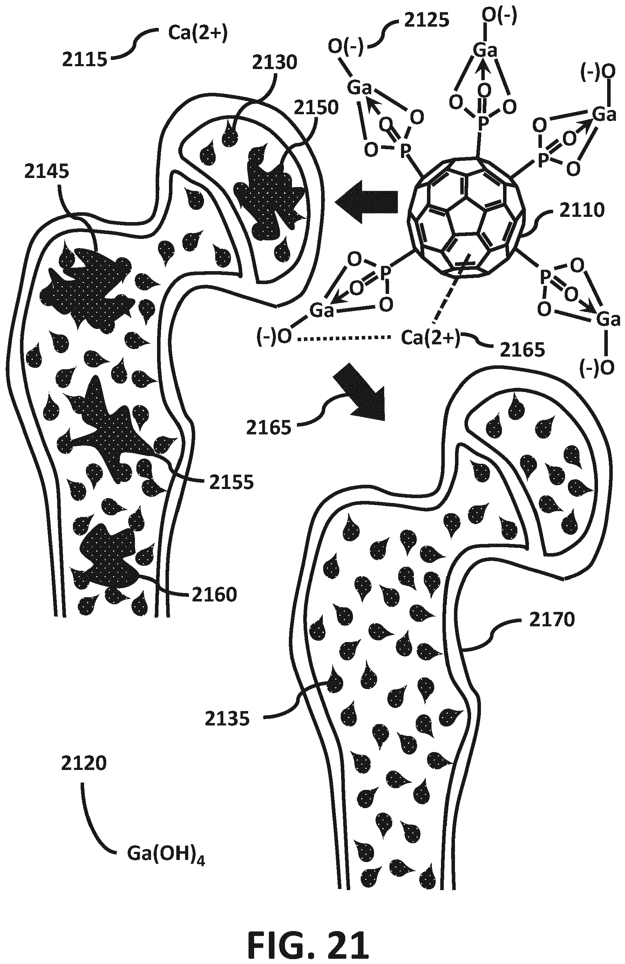

FIG. 21 is an illustration of FGP mediated bone density recovery from osteoporosis. Fullerene gallium phosphonate 2110 promotes calcium retention to treat osteoporosis by means of interposing between collagen and the calcium phosphate among bone mesenchymal cells. In the confined regions of the interior of bone marrow, the pH rises as calcium ions (Ca2+) 2115 are solvated. In addition, FGP donates some gallium hydroxide as Ga(OH)₄ 2120 which also causes the pH to rise. These effects cause the administered FGP 2110 to lose protons attached to at least some of the terminal hydroxyl groups 2125 at the interstitial spaces of the bone marrow 2130, 2135. Bones with osteoporosis become embrittled by the coalescence within their structure of ever larger cavities 2145, 2150, 2155, 2160. The negative charges expressed on FGP can cause a multiplicity of free calcium cations represented by 2165 to form aromatic-pi bonds with the carbon face of the fullerene group as indicated by the barbed dashed line, and the formation of ionic attraction to the negatively charged oxygen atoms of the gallium atoms indicated by the dotted line. The increase of the pH on gallium hydroxide release attracts and initiates the growth of osteoclast containing marrow tissues sufficiently to cause the gain of mesenchymal cell fecundity. The direction of this biochemical process is shown by the large black arrow 2165 to show the resulting bone 2170 is provided with restoration of calcium phosphate as hydroxyapatite to crystallize out onto the collagen and hyaluronic acid of the bone marrow 2135 to fill in the larger pores and voids and substantially reinforce the density of the marrow during and after FGP therapy, according to these teachings.

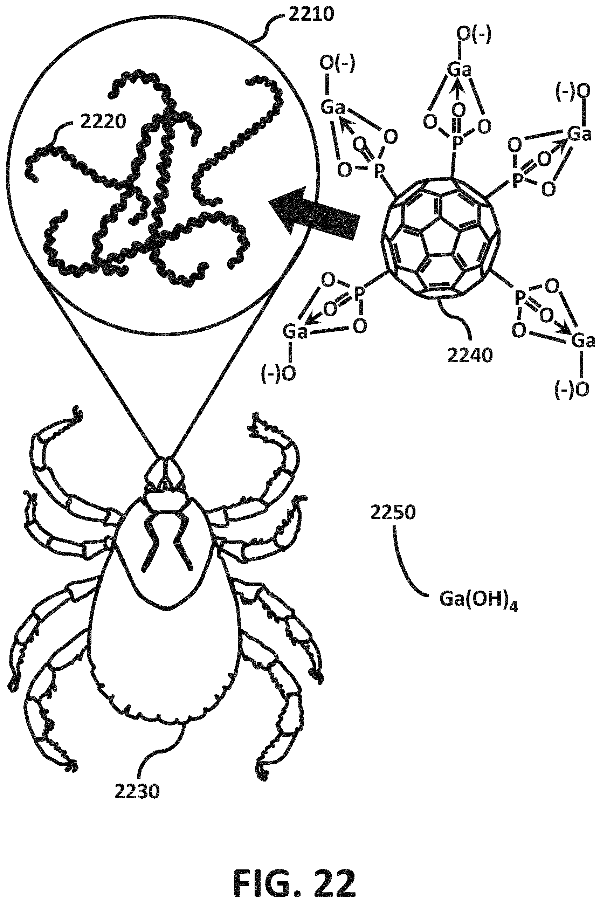

FIG. 22 is an illustration of the use of FGP to treat insect vectored diseases. Enlarged inset view 2210 magnifies the appearance of arthropod transmitted pathogens, here being the exemplary zoonotic spirochete bacteria 2220 such as *Borrelia burgdorferi,* or *Borrelia garinii,* well-known to cause long-term dermatological, neurological, musculoskeletal and cardiac pathologies. Such bacteria often carry viruses which inhabit the bacteria internally, wherein these are injected into the mammalian host by the bite of an insect vector, being the exemplary tick 2230. It is understood that the same or similar vectored transmission may be accomplished by the bite of other arthropods such as a flea, or a biting horsefly, and like insects. Of special concern is that these diseases are spreading exponentially with small incremental average global temperature increases which favor the proliferation of blood seeking and biting insects. Human patients are quite able to respond with antibodies to these pathogens over time, and several drugs and antibiotics help control these types of pathogens.

However, the Lyme Disease or Garin-Bujadoux-Bannwarth syndrome, and similar arthropod vectored zoonotic pathogens such as *Babesia venatorum* intraerythrocytic protozoa, primarily transmitted by hard-bodied (ixodid) ticks, are rarely able to become completely eradicated by modern drugs, which are unable to perform bacterial cell lysis without some form of prosthetic assistance to penetrate their protective proteins. This result leads to decades of recurrent inflammatory relapses with neurological and vascular damage from the sequestration of latent pathogens having resistance to antibiotics and antibodies. These types of microbes hide within difficult to treat regions of the human body that act as reservoirs. For example, some microbial reservoirs of latent pathogens may be the vitreous humor of the eye; another reservoir is the fibrinogen and fibrin micro-clots that may persist within the walls of the vasculature of the human body. An alternative way to kill living bacteria without the use of antibiotics is to remove their ability to respire. Bacterial respiration strongly depends on the presence of iron in the environment. Iron is present in the heme group of red blood cells. The pathogenic bacteria will mistakenly accept Ga3+ in place of Fe3+ in their environment. This is how gallium kills bacteria by interfering with bacterial respiration.

For this reason, traditional treatment and therapies for arthropod vectored pathogens require the assistance of fullerene gallium phosphonate nano surfactant 2240. The FGP donates gallium hydroxide 2250 under physiological conditions and pH, which are lethal to the arthropod vectored microbes 2220 trapped within fibrin bonded blood clots such as those illustrated in FIG. 14. The FGP releases gallium hydroxides Ga(OH)₄ 2250 which bind to the external glycan peptides of the spirochete bacteria while at the same time the fullerene group is well known to create a protein corona around itself, enabling the disruption of fibrin biopolymers. These properties unmask the entangled pathogenic bacteria for recognition by macrophages and antibodies, and provide Ga3+ as a substitute for Fe3+, which is a lethal substitution for bacteria while being a substantially benign substitution for the human patient. FGP nano surfactant penetrant enables the microbial release of the entrapped pathogens by disruption of polymeric fibrin for subsequent recognition and disposal of the gallium terminated bacteria by the immune system, according to these teachings.

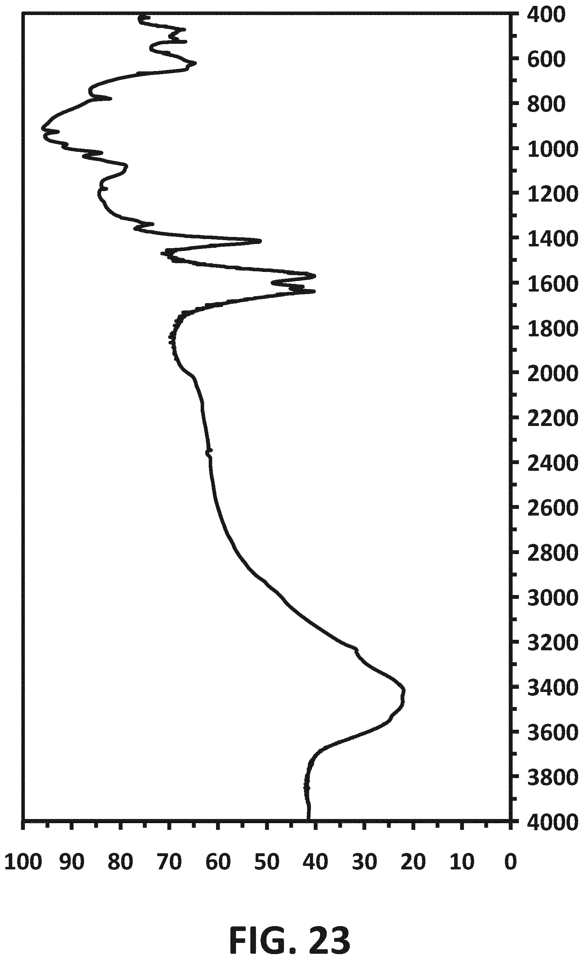

FIG. 23 shows experimental FTIR data for fullerene phosphonate. This sample tested by FTIR was prepared by a method of mixing, crushing, and consolidating under 7 metric tons of pressure, about 0.001 grams of analyte with 1 gram of a diluent solid material that is substantially transparent to infrared light, the diluent here being anhydrous potassium bromide (KBr), which then flows under pressure to form a translucent pellet of about 0.4 mm thickness. Spectral background subtraction in air using a pure KBr control pellet of the same mass and thickness was used to obtain a baseline instrument infrared spectral transmission response. This method is generally referred to as the 'KBr pellet' sample preparation method, and it is used hereinafter throughout for each FTIR experimental data collection and spectral analysis. The Fourier transform infrared spectrophotometer used herein to obtain the FTIR spectrum is a model RF6000 FTIR instrument manufactured by Shimadzu of Japan.

The weak absorbance from 2900 cm⁻¹ to 3500 cm⁻¹ indicates the presence of hydroxyl groups pendant from the fullerene phosphonate. The absorbance peak at 2359 cm⁻¹ can be ignored as this arises from local variations in the atmosphere of carbon dioxide. The absorbance at 1413 cm⁻¹ arises from the fullerene carbon to phosphorous bond (C60-P). The characteristic vibrations of the fullerene phosphonate group (CPO₃) are observed with absorbances for phosphorous to oxygen as P-O(-) bond stretching at 1022 cm⁻¹, and (P-OH) attributed to the 982 cm⁻¹, and 928 cm⁻¹ absorbances. The strong and sharp absorbance peak at 1570 cm⁻¹ is attributed to the presence of the double bonded phosphonyl (P=O) group. The bidentate or 'two-toothed' infra-red phosphonyl absorbance bands represent the bidentate pair of single bonded oxygen ligands to phosphorous (P-O) at 1639 cm⁻¹ and 1617 cm⁻¹ being of greater intensity than the double bonded (P=O) absorbance at 1570 cm⁻¹. The low intensity sharp and narrow rider peaks at 526 cm⁻¹ and 726 cm⁻¹ are typical of delocalized aromatic carbon (C-C) bonding vibrations of the fullerene group.

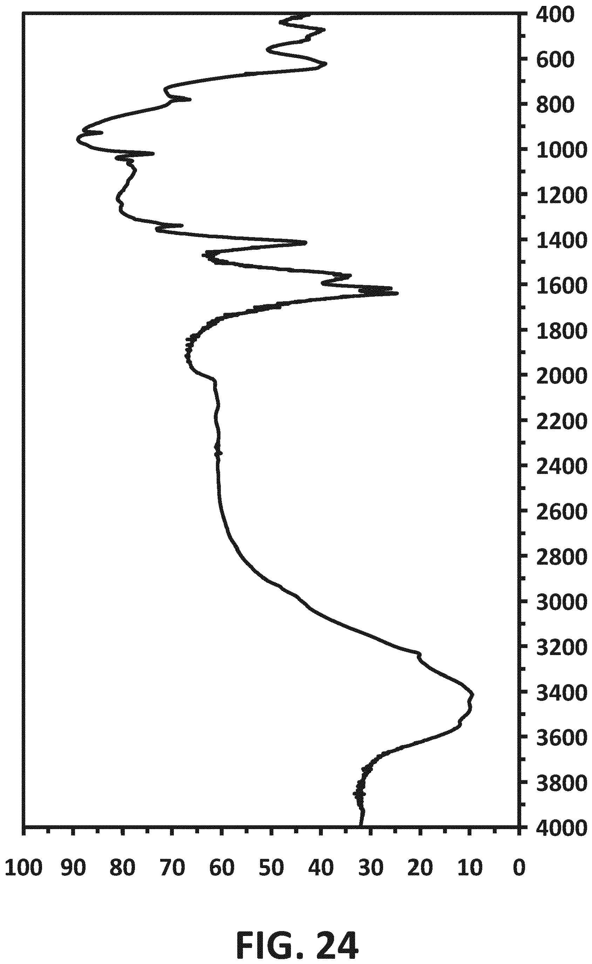

FIG. 24 shows experimental FTIR data for the molecular structures fullerene gallium phosphonate having hydrolyzed gallium atoms of Ga3+ valence reacted to the phosphonate groups. This sample underwent drying at 105C for 4 hours to remove trace liquid water that was used for processing. The absorbance at 1413 cm⁻¹ arising from the fullerene carbon to phosphorous bond (C60-P) is unchanged from the observation of fullerene phosphonate shown in FIG. 21. One of the characteristic vibrations of the phosphonyl (P=O) is now observed at 1060 cm⁻¹ indicating a shift from that of 1070 cm⁻¹ wherein the frequency change is attributed to the presence of an added gallium atom that serves to increase the proximal mass of the phosphonate functional group as gallium phosphonate. The intensity of this absorption is also significantly reduced, while promoting the bidentate or 'two-toothed' infra-red phosphonyl absorbances at 1639 cm⁻¹ and 1617 cm⁻¹ to significantly increased relative intensity by means of the geometric alignment of both of the single oxygen bonds with the gallium atom. The previously observed contribution of protons from the phosphonate (P-OH) absorbances at 982 cm⁻¹, and 928 cm⁻¹ in FIG. 20 are now completely absent for the fullerene gallium phosphonate infra-red spectrum, which is attributed to the replacement of the two oxygen bonds to hydrogen atoms with that of two oxygen bonds to one gallium atom in the functional group of (P-O-Ga). A significant new curvature of the baseline from 900 cm⁻¹ to 400 cm⁻¹ indicates a very broad and wide underlying absorbance contribution from the gallium with a strongest regional contribution being nearest to 400 cm⁻¹. These multiple indications are consistent with the achievement of those chemical changes characterizing fullerene gallium phosphonates according to these teachings.

As variations, combinations and modifications may be made in the construction and methods herein described and illustrated without departing from the scope of the invention, it is intended that all matter contained in the foregoing description or shown in the accompanying drawings shall be interpreted as illustrative rather than limiting. Thus, the breadth and scope of the present invention should not be limited by any of the above-described exemplary embodiments but defined in accordance with the foregoing claims appended hereto and their equivalents.

## Claims

1. An antimicrobial nanoparticle composition comprising:
a C60-buckminsterfullerene(110) bonded to gallium phosphonate to form a fullerene gallium phosphonate (310; 410; 520; 605; 1460; 2110; 2240).

2. The antimicrobial nanoparticle composition of claim 1 further comprising a solvent, wherein the fullerene gallium phosphonate (310; 410; 520; 605; 1460; 2110; 2240) is disposed in the solvent.

3. The antimicrobial nanoparticle composition of claim 2 wherein the solvent comprises a mixture of 70% glycerol and 30% polypropylene glycol by volume.

4. The antimicrobial nanoparticle composition of claim 1 further comprising a food grade solid carrier, wherein the fullerene gallium phosphonate (310; 410; 520; 605; 1460; 2110; 2240) is mixed with the food grade solid carrier, wherein the food grade solid carrier preferably includes baking powder, baking soda, or a powdered sweetener to make a food product.

5. The antimicrobial nanoparticle composition of claim 4 wherein the fullerene gallium phosphonate (310; 410; 520; 605; 1460; 2110; 2240) mixed with the food grade solid carrier is disposed in a gelatin capsule or formed into a tablet.

6. The antimicrobial nanoparticle composition of claim 1 further comprising a blood plasma with the fullerene gallium phosphonate (310; 410; 520; 605; 1460; 2110; 2240) dissolved therein.

7. The antimicrobial nanoparticle composition of claim 1 further comprising an organic radical group (1915; 1960) including a carbon atom that is bonded to at least one carbon atom of the C60 (110), wherein at least one gallium phosphonate (1920) is also bonded to the organic radical (1915; 1960).

8. The antimicrobial nanoparticle composition of claim 7 further comprising more than one gallium phosphonate substituted organic radicals bonded to the C60 (110).

9. The antimicrobial nanoparticle composition of any of the preceding claims for use in a method of curing, treating, or prophylactically avoiding cancer, valley fever, COPD, pneumonia, insect vectored diseases, and antibody-resistant bacterial infections in a subject, comprising the step of:
administering to the subject a pharmaceutically effective amount of a composition including a buckminsterfullerene (C60) bonded to gallium phosphonate to form a fullerene gallium phosphonate.

10. The antimicrobial nanoparticle composition of claim 9 wherein the composition includes a food grade solid carrier including baking powder or baking soda, and the fullerene gallium phosphonate is disposed in the food grade solid carrier, wherein the composition preferably comprises a tablet, capsule, pill, powder, granule, or a liquid containing a dosage of 50 mg to 1000 mg of fullerene gallium phosphonate.

11. The antimicrobial nanoparticle composition of claim 9 or 10 wherein administering the composition comprises:
administering an oral dosage including up to about 500 mg of the fullerene gallium phosphonate,
administration by an intravenous, intramuscular, subcutaneous, intrathecal, intraperitoneal, topical, nasal, or oral route,
administering an intramuscular, intravenous, or a subcutaneous dose of fullerene gallium phosphonate in an amount of from about 0.1 mg/Kg to about 5 mg/Kg, and/or
administering a volume of an inhalant in the form of an air dispersed nano aerosol, a water mist dispersed vapor, an air dispersed powder, an air dispersed dust, or an air dispersed aerosol.

12. A method of making an antimicrobial fullerene gallium phosphonate (310; 410; 520; 605; 1460; 2110; 2240), the method comprising:
reacting phosphorous acid (120) with C60-buckminsterfullerene (110) to produce fullerene phosphonate (130); and
reacting the fullerene phosphonate (130) with gallium metal (220) to form fullerene gallium phosphonate (310; 410; 520; 605; 1460; 2110; 2240).

13. A method of making an antimicrobial organic radical substituted bis-fullerene gallium phosphonate or tris-fullerene gallium phosphonate, the method comprising:
reacting phosphorous acid with a bis-C60 or a tris-C60 to produce bis-fullerene phosphonate or tris-fullerene phosphonate; and
reacting the bis-fullerene phosphonate or tris-fullerene phosphonate with gallium metal to form bis-fullerene gallium phosphonate or tris-fullerene gallium phosphonate.

14. The method of claim 12 or 13 wherein reacting the phosphorous acid with the C60, bis-C60, or tris-C60 is performed by reactive shear mixing.

15. The method of claim 12 or 13 wherein reacting the gallium metal with the fullerene phosphonate, bis-fullerene phosphonate or tris-fullerene phosphonate is performed by reactive shear mixing.
